# EUROPEAN PATENT APPLICATION

(11) **EP 2 289 938 A2**
(43) Date of publication of application: **02.03.2011**
(21) Application number: 10180402.9
(22) Date of filing: 13.06.2006
(51) Int. Cl.: C07K 16/12, C07K 14/31, A61K 39/395, A61K 39/085, A61P 31/04

(54) **Use of Panton-Valentine Leukocidin for treating and preventing staphylococcus infections**

(30) Priority: 13.06.2005 US 689526 P
(62) Divisional of application: 06784845.7
(71) Applicant: GlaxoSmithKline Biologicals S.A., 1330 Rixensart (BE)
(72) Inventor: Taylor, Kimberly Louise, Bethesda, MD 20814 (US); Fattom, Ali Ibrahim, Rockville, MD 20852 (US)
(74) Representative: Johnston, Caroline Louise

(57) **Abstract**

The present invention relates to compositions and methods for treating *Staphylococcus aureus* (*S. aureus*) infections. In particular, the present invention provides vaccines comprising a Panton-Valentine Leukocidin (PVL) antigen, antibodies which bind a PVL antigen and compositions containing the same, methods of making such compositions and methods for treating *S*. *aureus* infections, including those that are community acquired methicillin-resistant infections. The present invention also provides PVL antibodies, including PVL antibodies specific for a single PVL subunit, and PVL antigens, including conjugated and mutated PVL antigens.

## Description

### CROSS-REFERENCE TO RELATED PATENT APPLICATIONS

This application claims priority to U.S. provisional application serial number 60/689,526, which was filed on June 13, 2005, and which is incorporated herein by reference.

### FIELD OF THE INVENTION

The present invention relates generally to the treatment and prevention of bacterial infections. In particular, disclosed herein are compositions and methods for treating and preventing *Staphylococcus aureus* (*S. aureus*) infections, including community acquired methicillin resistant *S. aureus* (CA-MSRA) infections, using compositions comprising a Panton-Valentine Leukocidin (PVL) antigen or antibodies that specifically bind thereto. The present invention also relates generally to antibodies and antigens pertaining to the LukF-PV and LukS-PV proteins, to mutated versions of those proteins, and to fusion protein combinations of those PVL subunits.

### BACKGROUND

*Staphylococcus aureus* bacteria, often referred to as "staph," "*Staph. aureus,*" or "*S. aureus,*" are commonly carried on the skin or in the nose of healthy individuals. Approximately 20-30% of the population is colonized with *S*. *aureus* at any given time. These bacteria often cause minor infections, such as pimples and boils. However, *S*. *aureus* also causes serious and potentially deadly bacteremia, which is a medical condition characterized by viable bacteria present in the blood stream.

*S. aureus* expresses a number of virulence factors including capsular polysaccharides and protein toxins. PVL is a *S*. *aureus* protein belonging to a family of synergohymenotropic toxins, which damage membranes of host defense cells, white blood cells, and erythrocytes by the synergistic action oftwo non-associated classes of secretory proteins or subunits. Supersac et al., Infect. Immun. 61:580-7 (1993). This family of proteins includes alpha-hemolysin (alpha-toxin), beta-hemolysin, delta-hemolysin, gamma-hemolysin, Leukocidin (Luk) and PVL proteins (LukS-PV and LukF-PV).

PVL was first discovered by observing leukotoxic activity in *S*. *aureus.* Van der Velde, La Cellule, 10:401-9 (1894). Later Panton and Valentine were able to differentiate PVL from other hemolysins in the V8 strain from a subject with chronic fumuculosis. Panton P., Lancet, 222:506-8 (1932). Woodin then discovered that PVL is comprised of two subunits, LukS-PV and LukF-PV. Woodin AM., Biochem. J., 73:225-37 (1959) and Woodin AM., Biochem. J., 75:158-65 (1960).

PVL has been shown to be leukotoxic by pore induction for rabbit and human polymorphonuclear cells (PMNs) and macrophages. Finck-Barbancon et al., Biochim. Biophys. Acta, 1182:275-82 (1993). Purified PVL induces severe inflammatory lesions when injected intradermally in rabbits, leading to capillary dilation, chemotaxis, PMN infiltration, PMN karyorrhexis, and skin necrosis. Prevost et al., J. Med. Microbiol., 42:237-45 (1995) and Ward et al., Infect. Immun., 28:393-7 (1980). The leukotoxic and hemolytic activities of PVL involve sequential binding and synergistic association of the two PVL subunits. First, LukS-PV interacts with a membrane target (Colin et al., Infect. Immun. 62:3184-8 (1994)). Thereafter, the LukF-PV subunit binds the LukS-PV subunit. Woodin, AM and Wieneke AA, Biochem J., 105:1029-1038 (1967) and Colin *et al., supra.*

Although LukS-PV and LukF-PV are expressed in only a small percent of hospital-associated *S. aureus* isolates (Prevost et al., Infect. Immun., 63:4121-9 (1995)), PVL expression appears to be prevalent in community acquired methicillin resistant *S*. *aureus* (CA-MRSA) strains worldwide. Dufour et al., Clin. Infect. Dis., 35:819-24 (2002) and Vandenesch et al., Emerg. Infect. Dis., 9:978-84 (2003). Indeed, the emergence and spread of CA-MRSA has recently resulted in outbreaks of various diseases, including abscesses and furnunculosis (Lina et al., Clin. Infect. Dis., 29:1128-32 (1999) and Kazakova et al., N. Engl. J. Med., 352:468-75 (2005)), and severe toxemias such as necrotizing pneumonia. Francis et al., Clin. Infect. Dis., 40:100-7 (2005).

It is estimated that over 50% of strains of *S*. *aureus* in the United States are now methicillin-resistant. For example, in 1999, 54.5% of all *S*. *aureus* isolates reported in the National Noscomial Infections Surveillance System (NNISS) were methicillin resistant. The Centers for Disease Control estimate that in 2002 there were approximately 100,000 cases of hospital-acquired MRSA infections in the United States and the problem of these infections is only worsening. The rates of methicillin-resistance are even greater in certain Asian and European countries, (e.g., 72% MRSA rate in Japan; 74% in Hong Kong).

Accordingly, antibiotic resistant strains currently cause problems in treating *S*. *aureus* infections, and these problems will only become worse unless new treatment tools are developed. Therefore, there is a need for compositions and methods for treating *S*. *aureus* infections generally, and CA-MRSA infections in particular.

Prior attempts to make vaccines using PVL antigens suffered from significant drawbacks, including toxicity of the administered antigens and lack of efficacy. For example, Banffer & Franken, Path, Microbiol. 30: 16-74 (1967), reports the immunization of pregnant women with leukocidin toxoid (PVL) and its effect on antibody levels and incidence of mastitis. The authors found an increase in anti-leukocidin antibodies in the immunized subjects, but no statistically significant difference in the development of mastitis. The authors state that, out of 153 immunized subjects, 11 reported "soreness at the injection site with palpable axillary lymphglands (moderate)" and in "3 cases the reaction was considered severe."

Gladstone, Br. J. Exp. Path. 54: 255-59 (1973), comments on work done by the author and others that allegedly showed the therapeutic efficacy of purified leukocidin (comprising both LukF-PV and LukS-PV subunits) against staphylococcal infection, but acknowledges that "its use in healthy people ... is contraindicated by the variable local and general reactions often observed." The author reports the results of immunization with a purified leukocidin preparation, detoxified using formalin, noting that the immunization was well tolerated by 16 of 17, with the 17^{th} subject experiencing fever, malaise and vomiting. The author also reports considerable variability in anti-leukocidin antibody levels in immunized subjects. The paper did not investigate the prophylactic or therapeutic efficacy of the formalin-detoxified leukocidin preparation.

Ward & Turner, Infect. & Immun. 27: 393-97 (1980), report experiments conducted with preparations of LukF-PV and preparations of LukS-PV, although the purity of their preparations is not clear because they found that each preparation raised antibodies to both LukF-PV and LukS-PV, indicating either a lack of purity or antigenic cross-reactivity. The authors found that immunization with the LukF-PV preparation provided protection against subsequent challenge by the administration of LukF-PV, LukS-PV, or both, while immunization with the LukS-PV preparation did not provide protection against any of the challenges. However, the fact that the LukF-PV and LukS-PV challenges induced a toxic response indicates that each preparation was contaminated with the heterologous subunit because one subunit alone is not toxic.

With regard to the potential usefulness of anti-PVL antibodies, Gauduchon et al., J. Infect. Dis. 189: 346 (2004), found that commercial IVIG preparations could neutralize *S. aureus* PVL in vitro. The authors used purified recombinant PVL (rLukF-PV and rLukS-PV) to identify anti-PVL antibodies in commercial IVIG preparations. They found that pre-incubation of recombinantly produced antigen with IVIG inhibited cytotoxicity in an IVIG concentration-dependent manner. Similar results were found when culture supernatants of two different PVL-producing *S. aureus* strains were pre-incubated with IVIG. However, the authors did not demonstrate that the reported activity was due to anti-PVL antibodies per se, and did not control for the general immunomodulatory effect of IVIG.

Thus, there remains a need for compositions comprising PVL antigens and PVL antibodies that are useful in methods of treating and preventing *S*. *aureus* infection.

### SUMMARY

The present invention provides PVL antibody and antigen compositions, methods of making them, and methods of using them to prevent and treat *S. aureus* infection.

In one embodiment, the invention provides a antibody which specifically binds a Panton-Valentine Leukocidin (PVL) antigen of *S. aureus,* selected from the group consisting of (i) an antibody which specifically binds a LukF-PV subunit but does not specifically bind a LukS-PV subunit and (ii) an antibody which specifically binds a LukS-PV subunit but does not specifically bind a LukF-PV subunit. The antibody may be a polyclonal antibody or a monoclonal antibody. In one specific embodiment, the antibody is prepared by a process comprising (i) administering to a subject a composition selected from the group consisting of (a) a composition comprising a LukF-PV subunit as PVL antigen, and no LukS-PV subunit and (b) a composition comprising a LukS-PV subunit as PVL antigen, and no LukF-PV subunit and (ii) obtaining the antibody from the subject.

The invention also provides a composition comprising the antibody and a pharmaceutically acceptable carrier, and in one specific embodiment the composition is an IVIG composition, or a hyperimmune specific IVIG composition. In one particular embodiment, the antibody composition further comprises one or more antibodies to one or more other bacterial antigens, such as antibodies to one or more *S. aureus* antigens selected from the group consisting of *S*. *aureus Type* 5, *S. aureus* 8, *S. aureus* 336, *S*. *epidermidis* PS1, *S*. *epidermidis* GP1, α-toxin, lipoteichoic acid (LTA), and microbial surface components recognizing adhesive matrix molecule (MSCRAMM) proteins, and combinations thereof.

The invention also provides a method for neutralizing PVL-associated cytotoxicity in an individual, comprising administering to an individual a composition comprising the antibody. In one embodiment, the antibody specifically binds to LukS-PV. In another embodiment, the antibody specifically binds to LukF-PV.

The invention also provides a method of detecting PVL antigen in a sample, comprising contacting a sample with the antibody.

Another aspect of the invention relates to PVL antigen. In one embodiment, the invention provides a PVL antigen comprising a Panton-Valentine Leukocidin (PVL) antigen of *S. aureus* conjugated to another bacterial antigen. In one embodiment, the PVL antigen is selected from the group consisting of (a) PVL antigen comprising a LukF-PV subunit and no LukS-PV subunit and (b) PVL antigen comprising a LukS-PV subunit and no LukF-PV subunit. In one embodiment, the PVL antigen is selected from the group consisting of purified wild-type PVL antigens and recombinant PVL antigens. In one embodiment, the other bacterial antigen is selected from the group consisting of *S. aureus* Type 5, *S. aureus* Type 8, *S. aureus* 336, *S*. *epidermidis* PS1, *S*. *epidermidis* GP1, α-toxin, lipoteichoic acid (LTA) and microbial surface components recognizing adhesive matrix molecule (MSCRAMM) proteins. In another embodiment, the other bacterial antigen is another PVL subunit, and the PVL antigen comprises a conjugate selected from the group consisting of (i) a LukF-PV subunit conjugated to a LukS-PV subunit; (ii) a LukF-PV subunit conjugated to another LukF-PV subunit; and (iii) a LukS-PV subunit conjugated to another LukS-PV subunit. In one embodiment, the conjugate is a fusion protein or chemical conjugate.

In another embodiment, the invention provides a PVL antigen comprising a mutation in at least one of the LukF-PV or LukS-PV amino acid sequence, relative to its wildtype sequence, comprising at least one amino acid substitution, insertion, or deletion. In one embodiment, the mutation is selected from the group consisting of mutations that (i) prevent PVL binding to a cell membrane, (ii) prevent a stem or cytoplasmic extremity of a transmembrane domain from unfolding for LukS or F, (iii) block assembly of LukF-PV and LukS-PV, (iv) block Ca⁺² channel activity, (v) block activity of a PVL pore, (vi) alter the phosphorylation site of LukS-PV, (vii) disrupt membrane binding cleft of LukF-PV; (viii) create N-terminal deletions of the "amino latch" of PVL antigens, and (ix) create cysteine double mutants that prevent unfolding of pre-stem and insertion into the membrane. In one particular embodiment, the PVL antigen comprises a LukF-PV subunit comprising at least one mutation selected from the group consisting of (i) E191A, (ii) R197A, (iii) W176A, and (iv) Y179A. In another particular embodiment, the PVL antigen comprises a LukS-PV subunit comprising at least one mutation selected from the group consisting of (i) T28F, (ii) T28N, and (iii) T28D. In one embodiment, the PVL antigen is selected from the group consisting of (a) PVL antigen comprising a LukF-PV subunit and no LukS-PV subunit; (b) PVL antigen comprising a LukS-PV subunit and no LukF-PV subunit; (c) PVL antigen comprising a mutated LukF-PV subunit and wildtype LukS-PV subunit; (d) PVL antigen comprising a wildtype LukF-PV subunit and a mutated LukS-PV subunit; and (e) PVL antigen comprising a mutated LukF-PV subunit and a mutated LukS-PV subunit.

The invention also provides a composition comprising a PVL antigen of *S. aureus* and a pharmaceutically acceptable carrier. The PVL antigen may be any antigen described above. In one embodiment, the composition comprises a LukF-PV subunit and a LukS-PV subunit. In another embodiment, the composition comprises a LukF-PV subunit and no LukS-PV subunit or a LukS-PV subunit and no LukF-PV subunit. In one embodiment, the composition further comprises one or more additional bacterial antigens. In one particular embodiment, the one or more additional bacterial antigens is selected from the group consisting of *S*. *aureus* Type 5, *S. aureus* Type 8 and *S*. *aureus* 336, *S. epidermidis* PS1, *S*. *epidermidis* GP1, α-toxin, lipoteichoic acid (LTA) and microbial surface components recognizing adhesive matrix molecule (MSCRAMM) proteins.

The invention also provides an antibody that specifically binds to any of the PVL antigens described herein.

The invention also provides a method for treating or preventing *S. aureus* infection comprising administering to a subject in need thereof the composition comprising an antibody or antigen as described herein. The method may further comprise administering an agent selected from the group consisting of an anti-infective agent, an antibiotic, and an antimicrobial agent. In one embodiment, the antibiotic agent is selected from the group consisting of vancomycin, clindamycin and lysostaphin. In one embodiment, the *S*. *aureus* infection is selected from the group consisting of a community acquired methicillin resistant *S. aureus* (CA-MRSA) infection, a skin or soft tissue infection, necrotizing pneumonia, mastitis, necrotizing facsitis, Waterhouse Friderichsen Syndrome, CA-MRSA sepsism and infection by an *S. aureus strain* which expresses PVL antigen,

In one embodiment, the method further comprises administering one or more antibodies to one or more additional bacterial antigens. In one specific embodiment, the one or more antibodies are selected from the group consisting of antibodies to an *S. aureus* antigen selected from the group consisting of *S. aureus Type* 5, *S. aureus* Type 8, and *S. aureus* 336, *S*. *epidermidis* PS1, *S*. *epidermidis* GP1, α-toxin, lipoteichoic acid (LTA) and microbial surface components recognizing adhesive matrix molecule (MSCRAMM) proteins. In another embodiment, the method further comprises administering one or more additional bacterial antigens. In one specific embodiment, the one or more additional bacterial antigens are selected from the group consisting *S. aureus Type* 5, *S. aureus* Type 8, and *S. aureus* 336, *S*. *epidermidis* PS1, *S. epidermidis* GP1, α-toxin, lipoteichoic acid (LTA) and microbial surface components recognizing adhesive matrix molecule (MSCRAMM) proteins.

The invention also provides a method for making a hyperimmune specific IVIG preparation comprising (i) administering a PVL antigen to a subject, (ii) harvesting plasma from the subject, and (iii) purifying an immunoglobulin from the subject. In an alternative embodiment, the invention provides a method for making a hyperimmune specific IVIG preparation comprising (i) screening a subject that has not been administered a PVL antigen for high titres of anti-PVL antibodies, (ii) harvesting plasma from the subject, and (iii) purifying immunoglobulin from the subject. The invention also provides a composition comprising (i) an intravenous immunoglobulin (IVIG) composition comprising an antibody which specifically binds a Panton-Valentine Leukocidin (PVL) antigen of *S*. *aureus* and (ii) a pharmaceutically acceptable carrier, wherein the IVIG composition comprises an anti-PVL antibody titre that it at least two times greater than that found in normal IVIG.

Further aspects of the invention are described in more detail below.

### BRIEF DESCRIPTION OF THE DRAWINGS

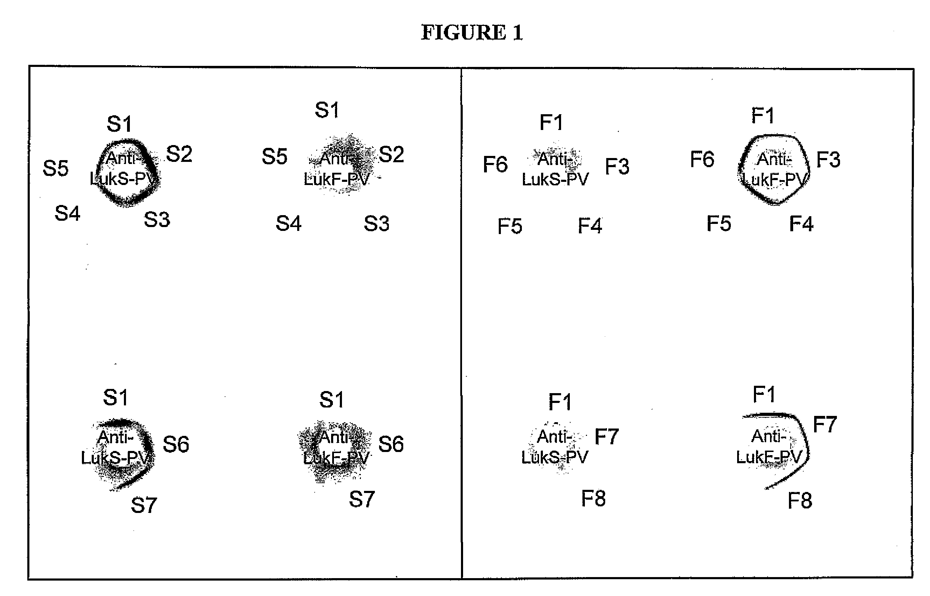

Figure 1. Immunodiffusion of rLukS-PV and rLukF-PV antigens with anti-LukS-PV and anti-LukF-PV rabbit antisera.

### DETAILED DESCRIPTION

The present invention provides compositions and methods for treating and preventing *S*. *aureus* infections, including CA-MSRA infections. The compositions comprise a PVL antigen, as defined and described in more detail below, or antibodies that specifically bind to a PVL antigen. The methods comprise administering a PVL antibody or PVL antigen composition according to the invention to a patient in need thereof.

In the discussion that follows, "a," "an," and "the" means "one or more," unless otherwise specified. In addition, where aspects of the invention are described with reference to lists of alternatives, the invention includes any individual member or subgroup of the list of alternatives and any combinations of one or more thereof.

### I. Compositions

### Panton Valentine Leukocidin (PVL) Antigen

The present invention provides compositions comprising a PVL antigen. As used herein, "PVL antigen" refers to an isolated and purified wild-type PVL antigen, a recombinant PVL antigen, a PVL antigen that comprises a LukS-PV subunit only or a LukF-PV subunit only, and a PVL antigen that comprises both a LukS-PV and a LukF-PV subunit. For example, a wild-type PVL antigen comprising LukS-PV and LukF-PV subunits can be purified from the *S*. *aureus* prototype V8 strain (ATCC 49775) using a series of chromatographic steps. Finck-Barbancon et al., Biochim. Biophys. Acta, 1182:275-82 (1993) and Prevost et al., Infect. Immun., 63:4121-9 (1995).

In accordance with one embodiment, the PVL antigen is a recombinant PVL antigen. As used herein, the term "recombinant PVL antigen" designates a PVL antigen made by recombinant DNA methodologies. Such recombinant DNA methodologies are well known in the art. Generally speaking, recombinant PVL antigen is free from other proteins and cell components with which wild-type PVL is associated in its native state (e.g., proteins and cell components present in *Staph.* cells).

In accordance with one embodiment, the PVL antigen is a purified PVL antigen. As used herein, the term "purified PVL antigen" designates a PVL antigen that has been as least partially separated from other proteins and cell components with which wild-type PVL is associated in its native state (*e.g.*, proteins and cell components present in *Staph.* cells).

In specific embodiments of the invention, preparations of a single PVL subunit are provided, such as a LukF-PV preparation that does not contain LukS-PV, or a LukS-PV preparation that does not contain LukF-PV. The purity of such preparations can be confirmed, for example, by demonstrating that antibodies raised against a LukF-PV preparation do not specifically bind to LukS-PV, or that antibodies raised against a LukS-PV preparation do not specifically bind to LukF-PV. In some embodiments, preparations comprising a single PVL subunit are obtained by recombinant expression of the single PVL subunit in a host that does not contain (and is not engineered to contain) a functional gene encoding the other PVL subunit.

In accordance with one aspect of the invention, LukF-PV and LukS-PV subunits are recombinantly expressed in *E*. *coli* cells and then purified from *E*. *coli* using a two-step column scheme that includes ion exchange chromatography (using, for example, an SP-sepharose column) followed by affinity chromatography (using, for example, a ceramic hydroxyapatite (CHT) column).

A PVL antigen as described herein also refers to a PVL antigen fragment, a LukS-PV subunit fragment and a LukF-PV subunit fragment. Fragments suitable for use in the present invention possess antigenic properties similar to wild-type PVL antigen. For example, a PVL antigen fragment, a LukS-PV subunit fragment and a LukF-PV subunit fragment are fragments that induce antibodies that specifically recognize wild-type PVL antigen.

A PVL antigen according to the present invention (including LukS-PV and/or LukF-PV subunits and PVL and subunit fragments) may comprise one or more amino acid insertions, substitutions or deletions in at least one of the LukS-PV subunit, the LukS-PV subunit, or both. For example, one or more amino acid residues within the LukF-PV or LukS-PV sequence can be substituted by another amino acid of a similar polarity, which acts as a functional equivalent, resulting in a silent alteration. Substitutes for an amino acid within the sequence may be selected from other members of the class to which the amino acid belongs. For example, nonpolar (hydrophobic) amino acids include alanine, leucine, isoleucine, valine, proline, phenylalanine, tryptophan and methionine. Polar neutral amino acids include glycine, serine, threonine, cysteine, tyrosine, asparagine, and glutamine. Positively charged (basic) amino acids include arginine, lysine and histidine. Negatively charged (acidic) amino acids include aspartic acid and glutamic acid. Alternatively, non-conservative amino acid alterations may be made, including the alterations discussed in more detail below in the context of detoxification. Thus, in one embodiment, a non-conservative amino acid change is made to the PVL antigen to detoxify the protein or stabilize the protein and prevent insertion into the membrane.

"PVL antigen" as used herein also refers to a PVL antigen that has undergone a modification, such as a modification that (i) prevent PVL binding to a cell membrane, (ii) prevent stem or cytoplasmic extremity of a transmembrane domain from unfolding for LukS-PV or LukF-PV, (iii) block assembly of LukF-PV and LukS-PV, (iv) block Ca⁺² channel activity, (v) block activity of a PVL pore, (vi) alter the phosphorylation site of LukS-PV, (vii) disrupt membrane binding cleft of LukF-PV; (viii) create N-terminal deletions of the "amino latch" of PVL antigens, or (ix) create cysteine double mutants that prevent unfolding of pre-stem and insertion into the membrane.

As described in more detail below, one or more of these modifications can be effected by methods including chemical treatment, conjugation, and mutations such as amino acid deletion or substitution.

In one embodiment, the PVL antigen is a detoxified. As used herein, a "detoxified" PVL antigen does not allow the influx of divalent cations through the cellular calcium channels of neutxaphils or influx of mono-valent cation through the PVL pore or formation of a PVL pore.

PVL toxicity on human polymorphonuclear neutrophils (PMNs) can be measured by techniques known in the art, such as light or fluorescent microscopy, flow cytometry, and flourimetry. See, Staali et al., J Membrane Biol. 162: 209-216 (1998), Meunier et al., Cytometry 21: 241-247 (1995), Werner et al., Infection and Immunity: 70: (3) 1310-1318 (2002). For example, the PVL antigen induces the opening of an existing cellular calcium channel on the PMN membrane. The opening of the calcium channel and subsequent calcium influx can be monitored with the use of fluorescent indicators, Fura2, Fluo3, Fluo4 or Calcium 3 and assays for measuring the influx of Ca+ into the cell using with DMSO for differentiation into the mature neutrophils (PMNs) have been established.

In addition, the PVL forms separate pores by the insertion of its subunits into the cellular membrane. The pore formation can be measured by the flux of monovalent cations into or out of the cell. Ethidium bromide, is also able to enter the cell through these pores and therefore, ethidium bromide can be used to track the influx of monovalent cations. When the ethidium bromide enters the cell it intercalates with the nucleic acid and results in fluorescent emission. Intracellular fluorescence can be detected visually using a fluorescent microscope or quantitatively using a fluorimeter. Likewise, fluorescent indicators such as PBFI (phosphate binding fluorescent indicator) and Na-Green, which chelate potassium and sodium, can be used to monitor formation of PVL pores.

In one embodiment, the PVL antigen is molecularly detoxified, which can be accomplished by methods known in the art, including primer extension on a plasmid template using single stranded templates by the original Kunkel method (Kunkel, TA, Proc. Acad. Sci., USA, 82:488-492 (1985)) or double stranded DNA templates (Papworth et al., Strategies, 9(3):3-4 (1996)), and by PCR cloning (Braman,J. (ed.), IN VITRO MUTAGENESIS PROTOCOLS, 2nd ed. Humana Press, Totowa, NJ (2002), Ishii et al., Meth. Enzymol., 293, 53-71 (1998), Kammann et al., Nucleic Acids Res., 17:5404 (1989), Hemsley et al, Nucleic Acids Res., 17:6545-6551 (1989), Giebel et al., Nucleic Acids Res., 18:4947 (1990), Landt et al., Gene, 96:125-128 (1990), Stemmer et al., BioTechniques, 13:214-220 (1992), Marini et al., Nucleic Acids Res., 21:2277-2278 (1993), and Weiner et al., Gene, 151:119-123 (1994)).

In another embodiment, the PVL antigen is detoxified by chemical means, *e.g*., by conjugating the PVL antigen to another molecule. This embodiment encompasses PVL antigen that is not detoxified by any means other than conjugation to another molecule. In another embodiment, the PVL antigen is conjugated to another antigen, such as another PVL antigen. For example, a LukF-PV subunit may be conjugated to another LukF-PV subunit or to a LukS-PV subunit. While not wanting to be bound by any theory, the inventors believe that conjugation of a LukS-subunit to a Luk-F subunit, directly or through a linker, may detoxify the antigen by preventing the antigen from folding into its toxic state, e.g., by preventing the S and F subunits from interacting in the manner required to exhibit toxicity.

In another embodiment, the PVL antigen is conjugated to another antigen, such as another bacterial antigen, including a gram-negative or gram-positive antigen, another *staphylococcal* antigen, and/or a bacterial polysaccharide. For example, a PVL antigen may be conjugated to one or more other *S*. *aureus* antigens, such as an antigen selected from the group consisting of *S. aureus* Type 5, *S*. *aureus* Type 8, *S*. *aureus* 336, *S*. *epidermidis* PS1, *S. epidermidis* GP1, α-toxin, LTA, MSCRAMMs, other protective antigens or toxins, and combinations thereof. In another embodiment the PVL antigen is detoxified via a mutation in at least one of the LukF-PV or LukS-PV amino acid sequence, comprising at least one amino acid substitution, insertion, or deletion.

A composition, such as a vaccine, may comprise one or both of a LukF-PV subunit and a LukS-PV subunit. In accordance with one embodiment, a composition, such as a vaccine, also comprises one or more *S*. *aureus* antigens, such as an antigen selected from the group consisting of *S. aureus* Type 5, *S*. *aureus* Type 8, *S. aureus* 336, *S*. *epidermidis* PS1, *S. epidermidis* GP1, α-toxin, lipoteichoic acid (LTA) and microbial surface components recognizing adhesive matrix molecule (MSCRAMM) proteins, other protective antigens or toxins, and combinations thereof. Thus, for example, the vaccine compositions of the present invention may comprise a PVL-Type 5 conjugate, a PVL-Type 8 conjugate, a PVL-Type 336 conjugate, a PVL-PS 1 conjugate, a PVL-GP1 conjugate, a PVL- α-toxin, conjugate, a PVL-LTA conjugate, or a PVL-MSCRAMM conjugate, where any of these conjugates comprise a PVL antigen.

In one embodiment, the PVL antigen is derivatized and linked to another bacterial antigen, such as another *S*. *aureus* antigens, such as an antigen selected from the group consisting of *S. aureus* Type 5, *S. aureus* Type 8, *S. aureus* 336, *S*. *epidermidis* PS1, *S*. *epidermidis* GP1, α-toxin, lipoteichoic acid (LTA) and microbial surface components recognizing adhesive matrix molecule (MSCRAMM) proteins, other protective antigens or toxins, and combinations thereof. For example, Type 5 or Type 8 antigen can be activated by 1-ethyl-3-(3-dimethylaminopropyl) carbodiimide (EDAC) to form cysteamine derivatives. PVL is modified with N-succinimidyl-3-(-2-pyridyldithio)propionate (SPDP) and then conjugated to the cysteamine derivative of T5 CP or T8 CP via thiol replacement. The resulting conjugates can be separated from the non-conjugated antigen by size exclusion chromatography.

In another embodiment, the PVL antigen is conjugated to a 336 antigen, for example, by activating the hydroxyl groups on the antigen are activated using cyanogen bromide or 1-cyano-4-dimethylamino-pyridinium tetrafluoroborate, and binding through a linker containing nucleophilic group(s) or without a linker, to PVL. *See, for example,* Kohn et al. FEBS Lett., 154: 209:210 (1993); Schneerson et al., J. Exp. Med., 152:361-376 (1980); Chu et al. Infect. Immun., 40:245-256 (1983); Kossaczka et al., Infect. Immun., 68:5037-5043 (2000). The resulting conjugates can then be separated from unconjugated antigen. An analogous method can be used to conjugate PVL antigen to LTA.

In another embodiment, the PVL antigen is conjugated to α-toxin (alpha-hemolysin), a pore-forming and hemolytic exoprotein produced by most pathogenic strains of *S*. *aureus.*

In yet another embodiment, the PVL antigen is conjugated to a PS 1 or GP1 antigen, for example, by modifying the *S*. *epidermidis* PS1 or GP1 with adipic acid dihydrazide (ADH) via an EDC-facilitated reaction to prepare adipic acid hydrazide derivative of PS1 (PS1_{AH}). The PVL antigen is then succinylated and the succinic derivative of PVL (PVLsuc) is conjugated to PS1_{AH}, which is mediated by EDC. An analogous method can be used to conjugate PVL antigen to LTA.

There are other conjugation methods known in the art, e.g., periodate oxidation followed with reductive amination, carbodiimide treatment, and other methods and/or their different combinations that can provide direct or indirect (through a linker) covalent binding of PS and protein carrier and thus yield the conjugate. For example PVL antigen can be conjugated to another protein by treating the PVL antigen and protein using a non-reversible homobifuncional cross-linking agent, such as Bis(Sulfosuccinimidyl)suberate (BS³), which rapidly reacts with primary amines. *See also* Partis et al., J. Prot. Chem. 2: 263-77 (1983). Regardless of the method used to conjugate the antigen to the carrier protein, the covalent binding of PS to protein carrier converts PS from a T cell independent antigen to a T cell dependent antigen. As a result, PS-protein conjugate would elicit PS-specific antibody response in immunized animals in contrast to no such response observed upon administering PS alone.

As discussed above, the present invention contemplates fusion proteins comprising LukS-PV and LukF-PV, or comprising two LukF-PV subunits or comprising two LukS-PV subunits. Such fusion proteins may be created recombinantly or by chemical conjugation.

In one embodiment, a fusion protein of the present invention is expressed using appropriately constructed DNA sequences. For example, a nucleic acid sequence encoding a LukF-PV subunit may be ligated directly or indirectly to a nucleic acid sequence encoding an LukS-PV subunit, e.g., one end of the LukF-PV nucleic acid may be joined directly to one end of LukS-PV nucleic acid, or the sequences encoding the subunits may be separated by a "linker" or "spacer" nucleic acid sequence. The invention includes fusion proteins comprising a LukF-PV subunit linked directly or indirectly at its 3'-end to a LukS-PV subunit. The invention also includes fusion proteins comprising a LukS-PV subunit joined directly or indirectly at its 3'-end to a LukF-PV subunit.

The present invention contemplates the recombinant expression of LukF-PV and LukS-PV subunits in the same or different constructs, in the same or different expression vector. Thus, for example, a DNA sequence encoding LukF-PV and a DNA sequence encoding LukS-PV may be present in a single construct that is operably linked to appropriate regulatory elements, e.g., to a promoter and terminator, for expression. Alternatively the present invention contemplates expression using two constructs, one for expressing LukF-PV and one for expressing LukS-PV. In such an embodiment, each subunit sequence may be operably linked to its own regulatory elements, for example, with different promoters driving the expression of each subunit. The expression constructs may be present in the same or different expression vectors. Hence, the present invention contemplates recombinantly transcribing a single mRNA that comprises sequences for both the LukS-PV and LukF-PV subunits, or recombinantly transcribing at least two mRNA transcripts, each of which encodes a given subunit. When a single expression vector is used, a single host cell is used for expression, and will produce both subunits. When more than one expression vectors are used, one or more host cells may be used for expression. For example, a single cell may be used for all vectors, or one cell may be used for each vector, or one cell may be used for one vector and another cell may be used for one or more vectors.

Thus, the invention contemplates multiple cell lines each of which recombinantly expresses a particular subunit. A subunit that is expressed from one cell line may be isolated and then joined to another subunit that has been expressed and isolated from another cell line. In this embodiment, the two subunits may be joined chemically, such as by chemical conjugation. Of course, the invention also contemplates the chemical conjugation of PVL subunits obtained by non-recombinant means, such as subunits of native PVL, or subunits that expressed from the genome of a cell or model *staphylococcus* system.

Regardless of whether a PVL fusion protein is created using recombinant techniques or chemical conjugation, either or both of the LukF-PV and LukS-PV subunits may comprise one or more amino acid mutations, including amino acid substitutions, insertions or deletions relative to the wildtype sequence, such as those described below. Thus, the PVL fusion protein may comprise (i) a mutated LukF-PV and a wild-type LukS-PV, (ii) a mutated LukS-PV and a wild-type LukF-PV, or (iii) a mutated LukF-PV and a mutated LukS-PV.

In some embodiments of the invention, the PVL antigen is detoxified by modifying the PVL antigen so as to contain a mutation in at least one amino acid in the LukF-PV or LukS-PV amino acid sequence. Exemplary mutations may prevent PVL binding to a cell membrane, prevent a "stem" or cytoplasmic extremity of a transmembrane domain from unfolding for LukS-PV or LukF-PV, block assembly of a LukF-PV subunit and/or a LukS-PV subunit, block Ca⁺² channel activity, block activity Of PVL pore, alter a phosphorylation site of LukS-PV, and/or disrupt membrane binding cleft of LukF-PV. Exemplary mutations may generate or eliminate internal disulfide bonds, generate or eliminate phosphorylation sites, or eliminate interactions between the LukF-PV and Luks-PV subunits. Mutations can include at least one point mutation, at least one amino acid deletion, or a combination thereof.

In one embodiment, Thr28 of the LukS-PV subunit is substituted with, for example, a leucine, phenylalanine, asparagine, aspartic acid, histidine or cysteine. Mutations at this position affect assembly of leukotoxins. *See* Guillet et al., J. Biol. Chem., 279:41028-41037 (2004).

In another embodiment, a mutation at Thr246 of LukS from gamma-hemolysin is made. This amino acid position has been described to be responsible for leukocytolytic activity of gamma hemolysin. *See* Nariya et al., FEBS Letters, 415:96-100 (1997), which is incorporated herein by reference in its entirety. A point mutation at the postulated phosphorylation site, Thr244, or a deletion of residues Thr240-Thr244 of LukS-PV could also be made, thus destroying the leukocytolytic activity of LukS-PV.

Other mutations contemplated herein include at least one point mutation and/or at least one deletion in the "stem" or cytoplasmic extremity of a transmembrane domain of LukF-PV, such as at Va1110, Va1114, Tyr116, Tyr118, Ile122, Ile124 and/or Leu128, and similar mutations in the stem of LukS-PV, including Va1103, Val105, Leu109, Tyr111, Ile113 and/or Phe117. One or more amino acid deletions between Leu128-Ser135, Ile124-Ser129, and/or Ser125-Leu128 are also encompassed by the present invention. These mutations enable the discoupling of Ca⁺² induction of pore forming activities of PVL. *See* Moussa et al., FEBS Letters, 461:280-286 (1999) and Werner et al., Infect. Immun., 70:1310-1318 (2002), which are incorporated herein by reference in their entirety.

Other mutations contemplated in the present invention include those that create N-terminal deletions of the "amino latch" of PVL antigens, such as deletions at Ala1-Val12 of LukF-PV, Ile124-Ser129 of LukF-PV and/or Asp1-Ile7 of LukS-PV, Phe117-Ser122. Additionally, mutations that create cysteine double mutants to make disulfide linkages between a beta-sandwich core and pre-stem to prevent unfolding of pre-stem and insertion into the membrane are also suitable for use in the present invention. For example, LukF-PV cysteine mutants such as Val13Cys-Lys136Cys; Asp43Cys-Tyr116Cys or Ser45Cys-Gly119Cys, or LukS-PV cysteine mutants such as Ile7Cys-Asn130Cys; and Asp38Cys -Ile113Cys; or similar mutants are contemplated in the present invention. For instance, the present invention contemplates mutations in the beta-sandwich contact region of LukS-PV. Hence, certain mutations in this region include, but are not limited to T28F, T28N, and T28D, e.g., the threonine at position 28 of LukS-PV or at the amino acid position that corresponds to position 28, is replaced by phenylalanine, asparagine, or aspartate. The present invention also contemplates mutations in the phosphorylation site of LukS-PV, which abolish or reduce phosphorylation at that site. Hence, one particular mutation of that region includes, but is not limited to T244A.

Other mutations contemplated in the present invention include those that disrupt the membrane-binding cleft of LukF-PV, e.g., in the postulated phosphatidyl choline binding cleft. For example, LukF-PV mutants at positions N173, W176, Y179, E191 and R197, are contemplated in the present invention. In this vein, one specific mutation contemplated by the present invention includes, but is not limited to E191A, e.g., the glutamate at position 191 of LukF-PV or at the amino acid position that corresponds to position 191, is replaced by alanine. Similarly, other specific LukF-PV mutations include N173A, W176A, R197A, and Y179A.

The invention contemplates PVL antigens with combinations of one or more mutations discussed above, such as, for example, a PVL antigen with a deletion of the amino latch and a point mutant at a phosphorylation site of LukS-PV.

### Compositions/Vaccines

The present invention provides compositions, including vaccines, comprising a PVL antigen and a pharmaceutically acceptable carrier. The PVL antigen may be any PVL antigen described above, including a purified wild-type PVL antigen or a recombinant PVL antigen. The PVL antigen may include a LukF-PV subunit, a Luk-S PV subunit, or both, or may comprise fragments of PVL, of the LukF-PV subunit, of the Luk-S PV subunit, or of both. The PVL antigen may be a modified and/or detoxified antigen, as described above, and may be conjugated to another PVL antigen or another molecule such as another bacterial antigen. The PVL antigen may include one or more of the mutations described above, such as two mutations.

Methods for making vaccines are generally known in the art. *See, for example,* Di Tommaso et al., Vaccine, 15:1218-24 (1997), and Fattom et al., Infect. and Immun. 58:2367-2374 (1990) and 64:1659-1665 (1996).

A vaccine according to the invention typically comprises a pharmaceutically acceptable carrier. A pharmaceutically acceptable carrier is a material that can be used as a vehicle for the *Staphylococcus* antigen because the material is inert or otherwise medically acceptable, as well as compatible with the active agent, in the context of vaccine administration. In addition to a suitable excipient, a pharmaceutically acceptable carrier can contain conventional vaccine additives like diluents, adjuvants and other immunostimulants, antioxidants, preservatives and solubilizing agents. For example, polysorbate 80 may be added to minimize aggregation and act as a stabilizing agent, and a buffer may be added for pH control. The vaccine formulation described herein allows for the addition of an adjuvant with relative ease and without distorting the composition.

In addition, the vaccine of the present invention may be formulated so as to include a "depot" component to increase retention of the antigenic material at the administration site. By way of example, in addition to an adjuvant (if one is used), alum (aluminum hydroxide or aluminum phosphate), QS-21, dextran sulfate or mineral oil may be added to provide this depot effect.

### Antibodies

The present invention also provides compositions comprising an antibody which specifically binds to a PVL antigen of *S. aureus* (a "PVL antibody"), such as any of the PVL antigens described above, formulated with a pharmaceutically acceptable carrier. The antibody composition of the present invention may comprise a monoclonal antibody, a polyclonal antibody, an antibody fragment, or a combination thereof.

A "PVL antibody," as described herein, refers to a full-length (*i.e.*, naturally occurring or formed by normal immunoglobulin gene fragment recombinatorial processes) immunoglobulin molecule (e.g., an IgG antibody) or an immunologically active (*i.e*., specifically binding) portion of an immunoglobulin molecule, including an antibody fragment. An antibody fragment is a portion of an antibody such as F(ab')₂, F(ab)₂, Fab', Fab, Fv, sFv and the like. Regardless of structure, an antibody fragment binds with the same antigen that is recognized by the full-length antibody, and, in the context of the present invention, specifically binds a PVL antigen. Methods of making and screening antibody fragments are well-known in the art.

A PVL antibody of the present invention may be prepared by a number of different methods. For example, PVL antibody may be obtained from subjects administered a PVL antigen, or from plasma screened for PVL antibody, as discussed in more detail below. In accordance with another embodiment, the PVL antibody is made by recombinant methods. Recombinant monoclonal antibodies can be made by techniques well-known in the art. Recombinant polyclonal antibodies can be produced by methods analogous to those described in U.S. Patent Application 2002/0009453 (Haurum et al.), using a PVL antigen as the immunogen.

A PVL antibody in accordance with the invention may be a murine, human or humanized antibody. A humanized antibody is a recombinant protein in which the CDRs of an antibody from one species; *e.g*., a rodent antibody, are transferred from the heavy and light variable chains of the rodent antibody into human heavy and light variable domains. The constant domains of the antibody molecule are derived from those of a human antibody. Methods for making humanized antibodies are well known in the art.

In one embodiment, an antibody that specifically binds to LukS-PV does not specifically bind to LukF-PV. In another embodiment, an antibody that specifically binds to LukF-PV does not specifically bind to LukS-PV. Thus, for example, an anti-LukS-PV antibody may not cross-react with LukF-PV and an anti-LukF-PV antibody may not cross-react with LukS-PV.

In some embodiments, an antibody of the present invention specifically binds to an epitope on a PVL subunit (*e*.*g*., LukF-PV or LukS-PV) that is present on PVL as it exists in a native state (*e*.*g*., its native folded state and/or its native state as complexed with the other PVL subunit), or to an epitope that is present on a fusion protein comprising that PVL subunit, *e.g*., by specifically binding to a conformational epitope on the native or fusion protein. In other embodiments, an antibody of the present invention specifically binds to one PVL subunit regardless of its three-dimensional configuration, *e.g.,* by specifically binding to a linear epitope.

In some embodiments, antibody of the present invention specifically binds to one or more of the mutated PVL antigens disclosed herein but does not cross-react with a wild-type version of that antigen. Hence, the invention includes antibodies that specifically bind to one of the Recombinant or chemically-conjugated fusion proteins described herein. Furthermore, an antibody of the present invention may specifically bind one or more of the mutated PVL antigens disclosed herein without cross-reacting with a wild-type version of that antigen. In some embodiments, a mutated PVL antigen is designed to have a mutation of a naturally-occurring PVL mutant or variant, and antibodies specific to that mutated PVL antigen are useful in diagnostic and therapeutic methods targeting the naturally-occurring PVL mutant or variant.

One method of the present invention entails administering one or more of such antibodies to an individual. In one embodiment, the antibody is an anti-LukS-PV antibody. In another embodiment, the antibody is an anti-LukF-PV antibody. In a further embodiment, one or both of such antibodies, *e.g*., the anti-LukF-PV antibody and/or the anti-LukS-PV antibody, are administered simultaneously or sequentially. Alternatively, only one antibody is administered to the individual.

The above-described antibodies can be obtained by conventional methods. For example, a PVL antigen (as defined above) can be administered to a subject and the resulting IgGs can be purified from plasma harvested from the subject by standard methodology. The PVL antigen used to obtain PVL antibody can be any PVL antigen described above. In one embodiment, the PVL antigen used to obtain PVL antibody is rendered non-toxic in accordance with the teachings above, including by mutation or conjugation. Alternatively, antibodies can be made recombinantly.

### Antibody Compositions

The invention includes antibody compositions suitable for administration, such as compositions comprising an antibody and a pharmaceutically acceptable carrier. The antibody compositions may be formulated for any route of administration, including intravenous, intramuscular, subcutaneous and percutaneous, by methods that are known in the art.

In one embodiment, the antibody composition is an IVIG composition. As described herein, "IVIG" refers to an immunoglobulin composition suitable for intravenous administration. "Specific IVIG" as used herein refers to an IVIG specific for one or more PVL antigens, such as any of the PVL antigens described above.

In accordance with one embodiment, the present invention provides a PVL antibody composition comprising an IVIG composition comprising an antibody which specifically binds a PVL antigen of *S*. *aureus,* such as any of the PVL antigens described above, and a pharmaceutically acceptable carrier.

In accordance with one embodiment, an IVIG composition comprising PVL antibody is obtained from plasma derived from donor subjects stimulated with a PVL antigen. In accordance with this embodiment, a PVL antigen (such as any described above) is administered to a subject, such as a human or other animal, including a mouse, to stimulate production of a PVL antibody. In one embodiment, the PVL antigen is administered as a vaccine. As a component of the vaccine, the PVL antigen is detoxified, such as by any means described above. Antibody which specifically binds a PVL antigen is then obtained from the subject by, for example, obtaining immunoglobulin from the plasma via conventional plasma-fractionation methodology.

In one specific aspect of this embodiment, the PVL antibody is obtained as a hyper-immune specific immunoglobulin (IGIV) preparation. A "hyper-immune specific IVIG" refers to an IVIG preparation containing high titres of PVL antibody. A hyperimmune specific IVIG composition can be obtained by administering a PVL antigen to a subject, harvesting plasma from the subject, and obtaining the hyper-immune specific IVIG from the plasma via conventional plasma-fractionation methodology. Alternatively, a hyperimmune specific IVIG composition can be obtained from plasma obtained from a subject that has not been administered a PVL antigen (*i.e.*, an unstimulated subject). In this embodiment, plasma from unstimulated subjects is screened for high titers of antibodies to a PVL antigen, including a PVL antigen that comprises only one of LukS-PV, LukF-PV, or both. In accordance with one embodiment, plasma is screened for PVL antibody titers that are 2-fold or more higher than the levels typically found in standard IVIG preparations, and such plasma is used to prepare a hyperimmune specific IVIG composition. Again, the subject can be either a human or animal.

The PVL antigen used to obtain the PVL antibody composition may be any PVL antigen described above, including a purified wild-type PVL antigen, recombinant PVL antigen, a PVL antigen that comprises one or both a LukF-PV subunit and a LukS-PV subunit, a PVL antigen with one or more amino acid insertions, substitutions, a PVL antigen with deletions in at least one of the LukF-PV or LukS-PV amino acid sequence, a modified PVL antigen, a fragment of a PVL antigen, or a dextoxified PVL antigen, including a PVL antigen detoxified by conjugation to another PVL antigen or another molecule.

In accordance with one embodiment, the PVL antibody composition of the present invention (including the IVIG and hyperimmune specific IVIG compositions) further comprises one or more antibodies to one or more *Staphylococcal* antigens, such as those described below. As described below, exemplary *S. aureus* antigens include Type 5, Type 8, and Type 336 *Staphylococcus* antigens. Exemplary *S*. *epidermidis* antigens include PS1 and GP1. For example, the composition may comprise antibodies to antigens selected from the group consisting of *S. aureus* antigens, such as an antigen selected from the group consisting of *S*. *aureus* Type 5, *S*. *aureus* Type 8, *S*. *aureus* 336, *S*. *epidermidis* PS1, *S. epidermidis* GP1, α-toxin, lipoteichoic acid (LTA) and microbial surface components recognizing adhesive matrix molecule (MSCRAMM) proteins, other protective antigens or toxins, and combinations thereof. Thus, in one embodiment, the IVIG composition of the present invention comprises at least one antibody that binds a PVL antigen and also binds a different *Staphylococcus* antigen, or at least one antibody that binds a PVL antigen and at least one antibody that binds a different *Staphylococcus* antigen.

### Additional Optional Antigen/Antibody Components

In addition to PVL antigens or antibodies described above, the PVL antigen or PVL antibody composition of the present invention may comprise additional antigens or antibodies, such as one or more *S*. *aureus* capsular polysaccharide antigens, such as the Type 5 and Type 8 antigens described in Fattom et al., Infec. and Immun., 58:2367-2374 (1990), and Fattom et al., Infec. and Immun., 64:1659-1665 (1996), or antibodies thereto. Additionally or alternatively, the composition may comprise the *S*. *aureus* 336 antigen described in U.S. Patent Nos. 5,770,208; 6,194,161; 6,537,559 or the *Staphylococcal* 336 CPS antigen described in U.S. Patents No. 5,770,208 and No. 6,194,161, or antibodies thereto.

Other *S*. *aureus* antigens are known in the art, *see, e.g.,* Adams et al., J. Clin. Microbiol., 26:1175-1180 (1988), Rieneck et al., Biochim. Biophys. Acta., 1350:128-132 (1977) and O'Riordan et al., Clin. Microbiol. Rev., 17: 218-34 (2004), and compositions comprising those antigens or antibodies thereto are also useful in the present invention.

Similarly, *S. epidermidis* antigens (or antibodies thereto) can also be used in accordance with the present invention. A *S. epidermidis* Type II antigen, also referred to a PS1, is disclosed in U.S. Patents No. 5,961,975 and No. 5,866,140. This antigen is an acidic polysaccharide antigen that can be obtained by a process that comprises growing cells of an isolate of *S. epidermidis* that agglutinates antisera to ATCC 55254 (a Type II isolate).

Yet another *Staphylococcus* antigen useful in the present invention is described in WO 00/56357. This antigen comprises amino acids and a N-acetylated hexosamine in an α configuration, contains no O-acetyl groups, and contains no hexose. It specifically binds with antibodies to a *Staphylococcus* strain deposited under ATCC 202176. Amino acid analysis of the antigen shows the presence of serine, alanine, aspartic acid/asparagine, valine, and threonine in molar ratios of approximately 39:25:16:10:7. Amino acids constitute about 32% by weight of the antigen molecule. This antigen, or antibodies thereto may be included in the PVL antigen (or PVL antibody) compositions of the present invention.

Another *Staphylococcus* antigen useful in the present invention is described in published U.S. patent application 2005/0118190, and is known as the *Staphylococcus epidermis* "GP1" antigen. That antigen is common to many coagulase-negtive strains of *Staphylococcus,* including *Staphylococcus epidermis, Staphylococcushaemolyticus,* and *Staphylococcushominis.* The antigen can be obtained from the strain of *Staphylococcus epidermis* deposited as ATCC 202176. This antigen, or antibodies thereto may be included in the PVL antigen (or PVL antibody) compositions of the present invention. Antigens also include those pertaining to lipoteichoic acid (LTA) and microbial surface components recognizing adhesive matrix molecule (MSCRAMM) proteins, and other protective antigens or toxins.

### Methods

### Methods for Treating and Preventing Bacterial Infection

The present invention provides methods for treating or preventing a *S. aureus* infection using compositions comprising a PVL antibody or a PVL antigen. A target patient population for the treatment and prevention methods described herein includes mammals, such as humans, who are infected with, or at risk of being infected by, bacterial pathogens, such a *S*. *aureus* (including CA-MSRA) or *S*. *epidermidis.*

In accordance with one embodiment, the invention provides a method for treating or preventing a *S. aureus* infection using compositions comprising a PVL antibody. In accordance with this method, a patient in need thereof is administered a composition that comprises an antibody which specifically binds a PVL antigen of *S. aureus* and a pharmaceutically acceptable carrier. The antibody composition may comprise any PVL antibody described above, and optionally may be an IVIG composition, a hyper-immune specific IVIG composition, a composition comprising recombinant PVL antibodies (including compositions comprising PVL antibody fragments), or a composition comprising humanized PVL antibodies.

The PVL antibody composition may be administered in combination with an anti-infective agent, an antibiotic, or an antimicrobial agent. Exemplary anti-infective agents include, but are not limited to vancomycin, clindamycin and lysostaphin. Exemplary antibiotics and antimicrobial agents include, but are not limited to penicillinase-resistant penicillins, cephalosporins and carbapenems, including vancomycin, lysostaphin, penicillin G, ampicillin, oxacillin, nafcillin, cloxacillin, dicloxacillin, cephalothin, cefazolin, cephalexin, cephradine, cefamandole, cefoxitin, imipenem, meropenem, gentamycin, teicoplanin, lincomycin and clindamycin. The dosages of these antibiotics are well known in the art. *See, for example,* MERCK MANUAL OF DIAGNOSIS AND THERAPY, § 13, Ch. 157, 100th Ed. (Beers & Berkow, eds., 2004). The anti-infective, antibiotic and/or antimicrobial agents may be combined prior to administration, or administered concurrently or sequentially with the disclosed IVIG composition.

In some embodiments, relatively few doses of PVL antibody composition are administered, such as one or two doses, and conventional antibiotic therapy is employed, which generally involves multiple doses over a period of days or weeks. Thus, the antibiotics can be taken one, two, or three or more times daily for a period of time, such as for at least 5 days, 10 days or even 14 or more days, while the PVL antibody composition is usually administered only once or twice. However, the different dosages, timing of dosages, and relative amounts of PVL antibody composition and antibiotics can be selected and adjusted by one of ordinary skill in the art.

The PVL antibody compositions of the present invention is suitable for treating community acquired methacillin resistant *S*. *aureus* (CA-MRSA) infections, including, but not limited to necrotizing pneumonia, mastitis, necrotizing fasciitis, Waterhouse-Friderichsen Syndrome, CA-MRSA sepsis, and skin and soft tissue infection. The appropriate dosage can be determined by one of ordinary skill in the art by routine methods. The dosage may depend on a number of factors, such as the severity of infection, the particular PVL antibody composition used, the frequency of administration, and subject details (such as age, weight and immune condition of the subject). In some embodiments, the dosage will be at least 50 mg PVL antibody composition per kilogram of bodyweight (mg/kg), including at least 100 mg/kg, at least 150 mg/kg, at least 200 mg/kg, at least 250 mg/kg, at least 500 mg/kg, at least 750 mg/kg, and at least 1000 mg/kg.

The frequency of dosage and number of dosages also depends on a number of factors, such as the severity of the infection and patient immune state. An appropriate dosing regimen, however, be determined by a skilled practitioner using routine methods known in the art. In some embodiments, the dose can be administered at lest once every other day, including at least once daily and at least twice daily. The number of doses needed to effectively treat the infection also can vary. For example, one, two, three, four, or more doses of the PVL antibody composition may need to be administered. A subject with a weakened immune system or particularly severe infection may require more dosages and/or more frequent dosing.

Also disclosed in the present invention are methods for treating and/or preventing a *S. aureus* infection using the antigen compositions described herein. Such methods comprise administering to a subject in need thereof a composition, such as a vaccine, that comprises a PVL antigen (as described above), and a pharmaceutically acceptable carrier. A target subject population for the treatment and prevention methods described herein includes mammals, such as humans, who are infected with, or at risk of being infected by, bacterial pathogens, such a *S. aureus.* Such methods include the prevention of CA-MRSA infections, including skin and soft tissue infections, necrotizing pneumonia, mastitis, nerconizing facsitis, Waterhouse Friderichsen Syndrome and CA-MRSA sepsis. As described above, a vaccine according to the present invention comprises a PVL antigen and a pharmaceutically acceptable carrier. In accordance with one embodiment, the PVL antigen is detoxified, as described above. In accordance with one specific embodiment, the PVL is detoxified by conjugation to another molecule, including by conjugation to another PVL antigen or another bacterial antigen.

The present invention also provides methods for treating and/or preventing a bacterial infection using an antigen composition, such as a vaccine, comprising a PVL antigen (as described above) conjugated to another bacterial antigen, such as a gram-negative or gram-positive bacterial antigen, such as a *staphylococcal* antigen or other bacterial polysaccharide. A target subject population for the treatment and prevention methods described herein includes mammals, such as humans, who are infected with, or at risk of being infected by, bacterial pathogens, such a *S. aureus.* In accordance with one embodiment, the PVL antigen is detoxified, as described above. In accordance with one specific embodiment, the PVL is detoxified by conjugation to another molecule, including by conjugation to another PVL antigen or another bacterial antigen.

An antigen composition or vaccine may be administered in conjunction with additional antigens, such as one or more *S. aureus* capsular polysaccharide antigens, such as the Type 5, Type 8, and 336 antigens described above, and/or other *S. aureus* known in the art. Additionally or alternatively, a composition or vaccine may be administered in conjunction with one or more *S. epidermidis* antigens, such as the PS1 antigen described above, or with any other *Staphylococcus* antigen, such as the antigen described in WO 00/56357 and the antigen described in published U.S. patent application 2005/0118190 (GP1) (discussed above). A composition or vaccine of the present invention also may comprise antigens such as α-toxin, lipoteichoic acid (LTA) or microbial surface components recognizing adhesive matrix molecule (MSCRAMM) proteins, or other protective antigens or toxins. The one or more additional antigens may be administered separately from the PVL vaccine composition or may be included in the PVL vaccine composition.

An antigen composition or vaccine may be administered in conjunction with an anti-infective agent, an antibiotic, and/or an antimicrobial agent, in a combination therapy as provided above. Also, a composition of vaccine according to the invention can be administered with or without an adjuvant. If an adjuvant is used, it is selected so as to avoid adjuvant-induced toxicity. A composition or vaccine according to the present invention may additionally comprise a β-glucan or granulocyte colony stimulating factor, in particular, a β-glucan as described in U.S. Patent No. 6,355,625, filed September 14, 1999 and issued March 12, 2002.

A therapeutically effective amount of the antigen composition or vaccine of the present invention can be determined by methods that are routine in the art. Skilled artisans will recognize that the amount may vary with the composition of the vaccine, the particular subject's characteristics, the selected route of administration, and the nature of the bacterial infection being treated. General guidance can be found, for example, in the publications of the International Conference on Harmonisation and in REMINGTON'S PHARMACEUTICAL SCIENCES, chapters 27 and 28, at pages. 484-528 (Mack Publishing Company 1990). A typical vaccine dosage may range from about 1µg to about 400 µg.

The composition or vaccine may be provided in any desired dosage form, including dosage forms that may be administered to a human intravenously, intramuscularly, subcutaneously, or percutaneously. The composition or vaccine or may be administered in a single dose, or in accordance with a multi-dosing protocol. Administration may be by any number of routes, including subcutaneous, intracutaneous, and intravenous. In one embodiment, intramuscular administration is used. The skilled artisan will recognize that the route of administration will vary depending on the bacterial infection to be treated and the composition of the vaccine.

### Methods for Identifying PVL Infection

The invention also provides method for screening samples for the presence of PVL antigen. In accordance with this aspect of the invention, any of the PVL antigens described above can be contacted with a sample, and binding between the antibodies and any PVL antigen present in the sample can be assessed. Antibody-based assays are well known in the art, and the invention contemplates both qualitative and quantitative assays using the antibodies of the invention to detect PVL antigen, including native PVL toxoid and any PVL antigen discussed above. The samples that can be tested for PVL antigen are not limited and include, for example, biological samples from a patient (such as blood or serum samples), cell culture supernatant samples, bacterial samples, and any other sample suspected of containing PVL antigen.

The invention contains the following embodiments:
1 An antibody which specifically binds a Panton-Valentine Leukocidin (PVL) antigen of *S. aureus,* selected from the group consisting of (i) an antibody which specifically binds a LukF-PV subunit but does not specifically bind a LukS-PV subunit and (ii) an antibody which specifically binds a LukS-PV subunit but does not specifically bind a LukF-PV subunit.
2 The antibody of embodiment 1, wherein the antibody is a polyclonal antibody.
3 The antibody of embodiment 1, wherein the antibody is a monoclonal antibody.
4 The antibody of embodiment 1, prepared by a process comprising:
   (i) administering to a subject a composition selected from the group consisting of (a) a composition comprising a LukF-PV subunit as PVL antigen, and no LukS-PV subunit and (b) a composition comprising a LukS-PV subunit as PVL antigen, and no LukF-PV subunit and
   (ii) obtaining the antibody from the subject.
5 The antibody of embodiment 4, wherein the PVL antigen is selected from the group consisting of purified wild-type PVL antigens and recombinant PVL antigens.
6 The antibody of embodiment 4, wherein the PVL antigen comprises a mutation in its amino acid sequence, relative to its wild-type amino acid sequence, comprising at least one amino acid substitution, insertion, or deletion.
7 The antibody of embodiment 6, wherein the mutation is at least one mutation selected from the group consisting of a mutation(s) that: (i) prevent PVL binding to a cell membrane, (ii) prevent a stem or cytoplasmic extremity of a transmembrane domain from unfolding for LukS or F, (iii) block assembly of LukF-PV and LukS-PV, (iv) block Ca⁺² channel activity, (v) block activity of a PVL pore, (vi) alter the phosphorylation site of LukS-PV, (vii) disrupt membrane binding cleft of LukF-PV; (viii) create N-terminal deletions of the "amino latch" of PVL antigens, and (ix) create cysteine double mutants that prevent unfolding of pre-stem and insertion into the membrane.
8 The antibody of embodiment 6, wherein the PVL antigen comprises a LukF-PV subunit comprising at least one mutation selected from the group consisting of (i) E191A, (ii) R197A, (iii) W176A, and (iv) Y179A.
9 The antibody of embodiment 6, wherein the PVL antigen comprises a LukS-PV subunit comprising at least one mutation selected from the group consisting of (i) T28F, (ii) T28N, and (iii) T28D.
10 The antibody of embodiment 4, wherein the PVL antigen is conjugated to another bacterial antigen.
11 The antibody of embodiment 10, wherein the PVL antigen is conjugated to an antigen selected from the group consisting of *S. aureus* Type 5, *S. aureus* Type 8, *S. aureus* 336, *S. epidermidis* PS1, *S. epidermidis* GP1, α-toxin, lipoteichoic acid (LTA) and microbial surface components recognizing adhesive matrix molecule (MSCRAMM) proteins.
12 A composition comprising the antibody of embodiment 1 and a pharmaceutically acceptable carrier.
13 The composition of embodiment 12, wherein the composition is an IVIG composition.
14 The composition of embodiment 12, wherein the composition is a hyperimmune specific IVIG composition.
15 The composition of embodiment 12, further comprising one or more antibodies to one or more other bacterial antigens.
16 The composition of embodiment 15, wherein the one or more antibodies is selected from the group consisting of antibodies to one or more *S. aureus* antigens selected from the group consisting of *S. aureus* Type 5, *S. aureus* 8, *S. aureus* 336, *S. epidermidis* PS1, *S. epidermidis* GP1, α-toxin, lipoteichoic acid (LTA), and microbial surface components recognizing adhesive matrix molecule (MSCRAMM) proteins, and combinations thereof.
17 A method for neutralizing PVL-associated cytotoxicity in an individual, comprising administering to an individual a composition comprising an antibody of embodiment 1.
18 The method of embodiment 17, wherein the antibody specifically binds to LukS-PV.
19 The method of embodiment 17, wherein the antibody specifically binds to LukF-PV.
20 A method of detecting PVL antigen in a sample, comprising contacting a sample with an antibody according to embodiment 1.
21 A composition comprising a PVL antigen of *S. aureus* and a pharmaceutically acceptable carrier.
22 The composition of embodiment 21, wherein the PVL antigen is conjugated to another bacterial antigen.
23 The composition of embodiment 22, wherein the other bacterial antigen is selected from the group consisting of *S. aureus* Type 5, *S. aureus Type* 8, *S. aureus* 336, *S. epidermidis* PS1, *S. epidermidis* GP1, α-toxin, lipoteichoic acid (LTA) and microbial surface components recognizing adhesive matrix molecule (MSCRAMM) proteins.
24 The composition of embodiment 22, comprising a PVL antigen conjugate selected from the group consisting of (a) a LukF-PV subunit conjugated to a LukS-PV subunit; (b) a LukF-PV subunit conjugated to another LukF-PV subunit; and (c) a LukS-PV subunit conjugated to another LukS-PV subunit.
25 The composition of embodiment 21, wherein the PVL antigen comprises a mutation in at least one of the LukF-PV or LukS-PV amino acid sequence, relative to its wild-type sequence, comprising at least one amino acid substitution, insertion, or deletion.
26 The composition of embodiment 25, wherein the PVL antigen comprises at least one mutation selected from the group consisting of mutations that (i) prevent PVL binding to a cell membrane, (ii) prevent a stem or cytoplasmic extremity of a transmembrane domain from unfolding for LukS or F, (iii) block assembly of LukF-PV and LukS-PV, (iv) block Ca⁺² channel activity, (v) block activity of a PVL pore, (vi) alter the phosphorylation site of LukS-PV, (vii) disrupt membrane binding cleft of LukF-PV; (viii) create N-terminal deletions of the "amino latch" of PVL antigens, and (ix) create cysteine double mutants that prevent unfolding of pre-stem and insertion into the membrane.
27 The composition of embodiment 25, wherein the PVL antigen comprises a LukF-PV subunit comprising at least one mutation selected from the group consisting of (i) E191A, (ii) R197A, (iii) W176A, and (iv) Y179A.
28 The composition of embodiment 25, wherein the PVL antigen comprises a LukS-PV subunit comprising at least one mutation selected from the group consisting of (i) T28F, (ii) T28N, and (iii) T28D.
29 The composition of embodiment 25, wherein the composition comprises PVL antigen selected from the group consisting of (a) PVL antigen comprising a mutated LukF-PV subunit and a wild-type LukS-PV subunit; (b) PVL antigen comprising a wild-type LukF-PV subunit and a mutated LukS-PV subunit and (c) PVL antigen comprising a mutated LukF-PV subunit and a mutated LukS-PV subunit.
30 The composition of embodiment 21, wherein the composition comprises a LukF-PV subunit and no LukS-PV subunit or a LukS-PV subunit and no LukF-PV subunit.
31 The composition of embodiment 21, further comprising one or more additional bacterial antigens.
32 The composition of embodiment 31, wherein said one or more additional bacterial antigens is selected from the group consisting of *S. aureus* Type 5, *S. aureus* Type 8 and *S. aureus* 336, *S. epidermidis* PS1, *S. epidermidis* GP1, α-toxin, lipoteichoic acid (LTA) and microbial surface components recognizing adhesive matrix molecule (MSCRAMM) proteins.
33 The composition of embodiment 31, further comprising one or more additional PVL antigens.
34 A PVL antigen comprising a Panton-Valentine Leukocidin (PVL) antigen of *S. aureus* conjugated to another bacterial antigen.
35 The PVL antigen of embodiment 1, wherein the PVL antigen is selected from the group consisting of purified wild-type PVL antigens and recombinant PVL antigens.
36 The PVL antigen of embodiment 34, wherein the other bacterial antigen is selected from the group consisting of *S. aureus* Type 5, *S. aureus* Type 8, *S. aureus* 336, *S. epidermidis* PS1, *S. epidermidis* GP1, α-toxin, lipoteichoic acid (LTA) and microbial surface components recognizing adhesive matrix molecule (MSCRAMM) proteins.
37 The PVL antigen of embodiment 34, comprising a conjugate selected from the group consisting of (i) a LukF-PV subunit conjugated to a LukS-PV subunit; (ii) a LukF-PV subunit conjugated to another LukF-PV subunit; and (iii) a LukS-PV subunit conjugated to another LukS-PV subunit.
38 The PVL antigen of embodiment 37, comprising a fusion protein or chemical conjugate of a LukF-PV subunit and a LukS-PV subunit.
39 The PVL antigen of embodiment 34, selected from the group consisting of (a) PVL antigen comprising a LukF-PV subunit and no LukS-PV subunit and (b) PVL antigen comprising a LukS-PV subunit and no LukF-PV subunit.
40 A PVL antigen comprising a mutation in at least one of the LukF-PV or LukS-PV amino acid sequence, relative to its wild-type sequence, comprising at least one amino acid substitution, insertion, or deletion.
41 The PVL antigen of embodiment 40, wherein the mutation is selected from the group consisting of mutations that (i) prevent PVL binding to a cell membrane, (ii) prevent a stem or cytoplasmic extremity of a transmembrane domain from unfolding for LukS or F, (iii) block assembly of LukF-PV and LukS-PV, (iv) block Ca⁺² channel activity, (v) block activity of a PVL pore, (vi) alter the phosphorylation site of LukS-PV, (vii) disrupt membrane binding cleft of LukF-PV; (viii) create N-terminal deletions of the "amino latch" of PVL antigens, and (ix) create cysteine double mutants that prevent unfolding of pre-stem and insertion into the membrane.
42 The PVL antigen of embodiment 40, wherein the PVL antigen comprises a LukF-PV subunit comprising at least one mutation selected from the group consisting of (i) E191A, (ii) R197A, (iii) W176A, and (iv) Y179A.
43 The PVL antigen of embodiment 40, wherein the PVL antigen comprises a LukS-PV subunit comprising at least one mutation selected from the group consisting of (i) T28F, (ii) T28N, and (iii) T28D.
44 The PVL antigen of embodiment 40, selected from the group consisting of (a) PVL antigen comprising a LukF-PV subunit and no LukS-PV subunit; (b) PVL antigen comprising a LukS-PV subunit and no LukF-PV subunit; (c) PVL antigen comprising a mutated LukF-PV subunit and wild-type LukS-PV subunit; (d) PVL antigen comprising a wild-type LukF-PV subunit and a mutated LukS-PV subunit; and (e) PVL antigen comprising a mutated LukF-PV subunit and a mutated LukS-PV subunit.
45 An antibody that specifically binds to a PVL antigen according to embodiment 34 or embodiment 40.
46 A method for treating or preventing *S. aureus* infection comprising administering to a subject in need thereof the composition according to any one of embodiments 17, 22, 25 or 31.
47 The method of embodiment 46, further comprising administering an agent selected from the group consisting of an anti-infective agent, an antibiotic, and an antimicrobial agent.
48 The method of embodiment 47, wherein the antibiotic agent is selected from the group consisting of vancomycin, clindamycin and lysostaphin.
49 The method of embodiment 46, wherein the *S. aureus* infection is selected from the group consisting of a community acquired methicillin resistant *S. aureus* (CA-MRSA) infection, a skin or soft tissue infection, necrotizing pneumonia, mastitis, necronizing facsitis, Waterhouse Friderichsen Syndrome, CA-MRSA sepsis and infection by an *S. aureus* strain which expresses PVL antigen.
50 The method of embodiment 46, further comprising administering one or more antibodies to one or more additional bacterial antigens.
51 The method of embodiment 50, wherein the one or more antibodies are selected from the group consisting of antibodies to an *S. aureus* antigen selected from the group consisting of *S. aureus* Type 5, *S.aureus* Type 8, and *S. aureus* 336, *S. epidermidis* PS1, *S. epidermidis* GP1, α-toxin, lipoteichoic acid (LTA) and microbial surface components recognizing adhesive matrix molecule (MSCRAMM) proteins.
52 The method of embodiment 46, further comprising administering one or more additional bacterial antigens.
53 The method of embodiment 52, wherein the one or more additional bacterial antigens are selected from the group consisting *S. aureus* Type 5, *S. aureus* Type 8, and *S. aureus* 336, *S. epidermidis* PS1, *S. epidermidis* GP1, α-toxin, lipoteichoic acid (LTA) and microbial surface components recognizing adhesive matrix molecule (MSCRAMM) proteins.
54 A method for making a hyperimmune specific IVIG preparation comprising (i) administering a PVL antigen to a subject, (ii) harvesting plasma from the subject, and (iii) purifying an immunoglobulin from the subject.
55 The method of embodiment 54, wherein the PVL antigen is selected from the group consisting of (a) PVL antigen comprising a LukF-PV subunit and no LukS-PV subunit; (b) PVL antigen comprising a LukS-PV subunit and no LukF-PV subunit, (c) PVL antigen comprising a mutated LukF-PV subunit and wild-type LukS-PV subunit; (d) PVL antigen comprising a wild-type LukF-PV subunit and a mutated LukS-PV subunit; (e) PVL antigen comprising a mutated LukF-PV subunit and a mutated LukS-PV subunit; and (f) PVL antigen conjugated to another bacterial antigen.
56 The method of embodiment 54, wherein the PVL antigen comprises a mutation in at least one of the LukF-PV or LukS-PV amino acid sequences, relative to the wild-type sequence, comprising at least one amino acid substitution, insertion, or deletion.
57 The method of embodiment 54, wherein the PVL antigen comprises a conjugate selected from the group consisting of (i) a LukF-PV subunit conjugated to a LukS-PV subunit; (ii) a LukF-PV subunit conjugated to another LukF-PV subunit; and (iii) a LukS-PV subunit conjugated to another LukS-PV subunit.
58 The method of embodiment 54, wherein the PVL antigen is conjugated to another bacterial antigen.
59 The method of embodiment 54, further comprising administering another bacterial antigen to the subject.
60 A method for making a hyperimmune specific IVIG preparation comprising (i) screening a subject that has not been administered a PVL antigen for high titres of anti-PVL antibodies, (ii) harvesting plasma from the subject, and (iii) purifying immunoglobulin from the subject.
61 A composition comprising (i) an intravenous immunoglobulin (IVIG) composition comprising an antibody which specifically binds a Panton-Valentine Leukocidin (PVL) antigen of *S. aureus* and (ii) a pharmaceutically acceptable carrier, wherein the IVIG composition comprises an anti-PVL antibody titre that is at least two times greater than that found in normal IVIG.

The invention is further described by reference to the following examples, which are provided for illustration only. The invention is not limited to the examples but rather includes all variations that are evident from the teachings provided herein.

### EXAMPLES

### Example 1. Generation of rLukF-PV and rLukS-PV Wild Type Clones

Genomic DNA was isolated from *S. aureus* strain deposited with the ATCC under Accession No. 49775, a PVL prototype strain that produces high levels of PVL, by using a protocol as per manufacturer (Promega) with slight modification (lysostaphin was added to the resuspension buffer).

Oligonucleotide primers were designed using the published sequences of PVL genes (GenBank accession numbers X72700 and AB006796) to bracket the LukF-PV and LukS-PV genes, separately. The forward primers were designed to eliminate the putative signal peptides and incorporate and NcoI site. The ATG of the NcoI site was designed to serve as the start codon for translation, eliminating the addition of vector encoded N-terminal amino acids. The reverse primers were designed to incorporate a BamHI site immediately downstream of the stop codon. The *luks*-PV and *lukf*-PV genes were amplified by PCR from *S. aureus* ATCC 49775 using standard PCR amplification conditions. The PCR products were cloned into pTrcHisB using the NcoI and BamHI sites. In addition, the NcoI-BamHI insert containing the *luks-PV* and *lukf-PV* genes were subsequently subcloned into pET28 (Novagen).

### Example 2. Generation of rLukF-PV and rLukS-PV Fusion Protein Clones

PCR cloning techniques are used to construct a PVL fusion protein, a human-engineered protein that is encoded by a nucleotide sequence made by a splicing together the *lukf-PV* and *luks-PV* genes. The PVL subunits are covalently attached through a short amino acid linker with the configuration rLukF-PV-aa linker-rLukS-PV or rLukS-PV-aa linker-rLukF-PV. This fusion protein is non-cytotoxic, because the two subunits are not able to assemble and/or interact in the manner need to exhibit toxicity, *e.g.*, in the manner needed to insert correctly into the leukocyte membrane. The fusion protein is useful for stimulating antibodies in a host to both subunits of PVL (e.g., LukS-PV and LukF-PV) and to the PVL toxin as a whole.

### Example 3. Generation of rLukF-PV and rLukS-PV Mutant Clones

The following mutants were constructed using the QuickChange mutagenesis kit using the protocol described by the manufacturer (Stratagene) and pTrcHisBLukF-PV and/or pTrcHisBLukS-PV as a template:
rLukF-PV mutants: ΔI124-S129; E191A; N173A; R197A; W176A, and Y179A.
rLukS-PV mutants: ΔD1-I17; ΔF117-S122; T28D; T28F; T28N, and T244A.

The person of ordinary skill in the genetics arts understands that this nomenclature is standard terminology. That is, "ΔI124-S129" means that the region terminated by isoleucine at position 124 and serine at position 129 is deleted ("Δ") in an rLukF-PV mutant. Similarly, "ΔD1-I17" indicates that residues 1 (aspartate) to 17 (isoleucine) are deleted in an rLukS-PV mutant. Likewise, the skilled person knows that "E191A" means that the glutamate at position 191 is replaced by alanine, that "N173A" indicates an rLukF-PV mutant has an alanine (A) at position 173 instead of the naturally-occurring asparagine (N), and that "R197A" means that the arginine (R) at position 197 is substituted for an alanine (A); and so on.

Other specifically contemplated mutants include:
rLukF-PV mutants: a double mutant V12C/K136C to generate internal disulfide bonds; ΔW176; ΔG175-G177; ΔR197 and ΔS196-Q198.
rLukS-PV mutants: T244A or ΔT244 to eliminate a phosporylation site; a double mutant I7C/N130C or a double mutant D38/I113C to create a stable disulfide bond, ΔT28; ΔV27-Q29; ΔG115-G124, and other double mutants such as T28D/AD1-I7.

All constructs were transformed into *E. coli* GC10 cells using the manufacturer's protocol (Gene Choice). Sequencing was performed using ABI PRISM Dye Terminator Cycle Sequencing. All clones with the correct sequence were transformed into *E. coli* GC10 or *E. coli* BL21(DE3) pLysS for expression.

### Example 4. Expression and Purification of rLukS-PV and rLukF-PV

### Wild Type and Mutant Antigens

In shake flasks, the *E. coli* strain GC10 or BL21(DE3) pLysS containing the rLukS-PV or rLukF-PV plasmid was cultured in selective medium at 37°C until mid-log phase and induced using final concentration of 1 mM isopropyl-beta-D-thiogalactopyranoside (IPTG) for 2-3 hours. The cells were harvested by centrifugation. Analysis of the shake-flask cultures by SDS-PAGE and Western blot analysis showed a band at approximately 33-34 kDa that was not evident prior to induction.

The pelleted cells were resuspended in cell lysis buffer (20 mM Na₂HPO₄, 50 mM NaCl, 5% glycerol, pH 6.5), and treated with 2 mg/g lysozyme at room temperature, followed by sonication with a Misonix sonicator. The supernatant of cell lysate was collected by centrifugation. The soluble protein was loaded on a cation exchange column pre-equilibrated with cell lysis buffer. The bound LukS-PV or LukF-PV protein was eluted with a linear gradient of 50 to 500 mM NaCl in 20 mM Na₂HPO₄, 5% glycerol, pH 6.5 buffer. The rLukS-PV or rLukF-PV containing fractions were pooled and applied on a ceramic hydroxyapatite column. The pure rLukS-PV or rLukF-PV was eluted from a linear gradient of 50 mM NaCl to 750 mM NaCl in 20 mM Na₂HPO₄, 5% glycerol, pH 6.8 buffer.

rLukF-PV and rLukS-PV recombinant proteins and mutants have been purified using this same methodology and found to be highly pure (∼33 or 34kDa single band for rLukF-PV and rLukS-PV, respectively; >95% pure by SDS-PAGE/Coomassie Blue staining). For western blot analysis, proteins were transferred to a polyvinylidene fluoride (PVDF) membrane and were processed using standard procedures known in the art using primary monoclonal antibody to rLukF-PV or rLukS-PV. Blots confirmed the presence of rLukS-PV and rLukF-PV antigens with a band roughly at ∼33-34 kDa. In addition, N-terminal sequencing of rLukS-PV and r LukF-PV confirmed the presence of the *lukS-PV* and *lukf-PV* gene products.

### Example 5. Production and characterization of rLukF-PV or rLukS-PV

### Polyclonal Antibodies

rLukS-PV or rLukF-PV (50 µg each) were injected into New Zealand White rabbits with adjuvant (CFA followed by IFA) at a 1:1 ratio 3 times 2 weeks apart. LukS-PV antiserum recognized rLukS-PV as an identical antigen in an immunodiffusion assay against the antigen, while rLukF-PV antiserum recognized LukF-PV. rLukS-PV or rLukF-PV did not react with the heterologous antisera. This indicates that neither the rLukS-PV vaccine or the rLukF-PV vaccine generated antibodies that were cross-reactive with the heterologous protein subunit. Thus, the vaccine of the present invention is useful for obtaining anti-LukS antibodies that do not cross-react with LukF-PV, and anti-LukF antibodies that do not cross-react with LukS-PV.

Positive bleeds were combined and IgGs were purified on a protein G column. Purified anti-PVL IgG were used in animal models, as described below.

### Example 6. Immunochemical Analysis of rLukF-PV or rLukS-PV Antigens

Double immunodiffusion was carried out to determine the specificity of the LukS-PV and LukF-PV antisera, as well as to determine the antigenicity of the PVL subunit antigens. Briefly, 10 µl/well of 200 µg/ml each PVL antigen (outside wells) and 10 µl/well of antiserum (center well) were loaded in 1% agarose gels and allowed to diffuse overnight in a humid environment. The agarose gels was then washed in PBS and pressed for three consecutive times, then dried and stained with Coomoassie blue. The gels were analyzed for precipitin bands, which are formed when antigen and antibody react and form an immune complex.

As shown in Figure 1, rLukF-PV antigens reacted with a single precipitin band with anti-LukF-PV antibodies, while rLukS-PV antigens did not cross-react with this antiserum. Similarly, rLukS-PV antigens reacted with a single precipitin band with anti-LukS-PV antibodies, while rLukF-PV antigens did not cross-react with these antibodies. Thus the antibodies are specific to the homologous PVL antigens.

The following antigens were tested:

| | |
|---|---|
| S1: rLukS-PV (wildtype) | F1: rLukF-PV (wildtype) |
| S2: rLukS-PV ΔD1-I17 | F3: rLukF-PV ΔI124-S129 |
| S3: rLukS-PV ΔF117-S122 | F4: rLukF-PV E191A |
| S4: rLukS-PV T28F | F5: rLukF-PV N173A |
| S5: rLukS-PV T28N | F6: rLukF-PV R197A |
| S6: rLukS-PV T28D | F7: rLukF-PV W176A |
| S7: rLukS-PV T244A | |

As demonstrated in Figure 1, all mutant rLukF-PV and rLukS-PV antigens were observed to share a line of identity with the homologous wild type recombinant subunit, showing that the mutant proteins are antigenically identical with the homologous wild type protein. For example wild type rLukS-PV, rLukS-PV ΔD1-I17, rLukS-PV ΔF117-S122, rLukS-PV T28F, rLukS-PV T28N, rLukS-PV T28D, and rLukS-PV T244A are each reactive with anti-LukS-PV antibodies and share a line of identity. Additionally, wild type rLukF-PV, rLukF-PV ΔI124-S129, rLukF-PV E191A, rLukF-PV N173A, rLukF-PV R197A, rLukF-PV W176A and rLukF-PV Y179A are reactive with anti-LukF-PV antibodies and share a line of identity.

Quantitative ELISA was performed on both anti-LukF-PV and anti-LukS-PV antibodies, demonstrating that there is no cross-reactivity for either PVL subunit against the heterologous antiserum. This data confirmed that the rLukF-PV and rLukS-PV antigens described herein are non-crossreactive PVL antigens.

### Example 7. LukS-PV and LukF-PV Hybridoma Production (Monoclonal Antibodies)

BALB/c mice were immunized with either rLukS-PV or rLukF-PV. Immunized splenocytes were collected from mice of the respective studies in separate procedures and fused to Sp2/O myeloma cells, in different experiments, using 50% polyethylene glycol. The fused cells were resuspended in a selection medium, seeded into 96-well tissue culture plates and incubated under humidified conditions in a 37°C incubator with 8% CO₂. Supernatants of growing cultures were screened on ELISA plates coated with purified antigens, representative of the respective immunogens (rLukS-PV or rLukF-PV), for monoclonal antibody (MAb) secretors. ELISA positives were re-screened for cross-reactivity on rLukS-PV and rLukF-PV antigens to verify the specificity of secreted MAbs before cloning experiments were performed to establish MAb secreting colonies generated from single cells. Seed stocks were generated from mass cultures established from these clones that were also used to produce mouse ascites fluid from which purified MAbs were prepared and further characterized.

### Example 8. Characterization ofLukS-PV and LukF-PV Monoclonal Antibodies

All monoclonal antibodies (MAbs) to each of the PVL protein subunits were shown to be of the IgG1 kappa sub-class. Supernatants of 10 MAbs each of LukS-PV and LukF-PV were tested in ELISA assays for cross-reactivity to rLukS-PV, and rLukF-PV antigens coated on to an ELISA plate. All MAbs were specific to their homologous antigens (LukS MAbs bind to rLukS-PV antigen only, while LukF MAbs bind to rLukF-PV antigen only). All MAbs were tested for binding to rLukS-PV and rLukF-PV proteins in Western blot assays. Again, each MAb was demonstrated to specifically bind to its antigen and demonstrated no cross-reactivity for the other antigen.

### Example 9. In Vitro Determination of PVL Activity by Calcium Influx Assay

HL-60 cells (ATCC CLL-240; Gallagher et al., Blood 54: 713-33 (1979)) were seeded at 2x10⁵ cells/ml and passaged after 6 or 7 days when the cell density reached approximately 1x10⁶ cell/ml. HL-60 cells were differentiated as follows: cell counts were performed to determine cell number and viability using Tryan Blue, DMSO was added to 1.25% in the cell culture media, and the cells were diluted to 2x10⁵ per ml in the cell culture media containing DMSO. The cells were cultured in a CO₂ incubator at 37°C/8% CO₂ for 6 to 7 days, after which cell counts were performed and the cell densities were determined to be approximately 1 x 10⁶ per ml.

Differentiated HL-60 cells were loaded with 10 µM Fluo-4 and 0.1% Pluronic acid F-127 for 30 min at room temperature. After incubation the cells were washed twice in HBSS / HEPES / Probenecid, and cells were adjusted to 6 x 10⁶ cell/ml in HBSS / HEPES / Probenecid, and added to each well of a 96-well black wall/clear bottom Costar microtiter. Five µL of 20 mM CaCl₂ was then added, followed by the subsequent addition of 25 µL of rLukS-PV and/or 25 µL rLukF-PV or buffer control. The cytotoxicity of PVL to HL-60 cells was determined by measuring the change in intracellular calcium as determined by change in fluorescence using Tecan's Safire2 monochrometer based microplate detection system.

An influx of calcium into the HL-60 cells as determined by change in fluorescence was detected only when both rLukS-PV and rLukF-PV were present, demonstrating that both PVL subunits are required for *in vitro* cytoxicity. One PVL subunit alone did not show an increase in florescence, thus demonstrating that rLukF-PV and rLukS-PV alone are non-cytotoxic and can be used individually as antigens for a vaccine without requiring detoxification.

Using the calcium influx assay under the conditions described above, a selection of the rLukS-PV and rLukF-PV mutants described above were evaluated for activity as compared to wild type proteins, using a mutant in concert with the heterologous wild type protein. For example, mutant rLukS-PV proteins were combined with wild type rLukF-PV, or mutant rLukF-PV proteins were combined with wild type rLukS-PV, and the activities of the mutant/wild-type combinations were compared to the activity of wild type rLukF-PV and rLukS-PV. Not all mutants were found to result in inactive complexes; however, several mutants did result in inactive forms (<10% of wild type activity) as determined by the calcium influx assay. See Tables 1 and 2 below. These included the LukF-PV mutants designed to have a mutation in the phosphatidyl choline binding cleft (*e.g*., E191A R197A, W176A and Y179A) and LukS-PV mutants T28F, T28N, and T28D.

As described above, any non-cytotoxic mutant (include those with point mutations, deletions, truncations, or doubly detoxified with two or more such mutations) of one subunit can be used with the wild type form of the heterlogous subunit to create a non-toxic stimulating antigen or vaccine. Additionally, mutants of both subunits can be used. In either case, the vaccine will induce antibodies to both LukS-PV and LukF-PV.

The non-toxicity of a fusion protein or chemical conjugate comprising LukF-PV and LukS-PV can be confirmed using the calcium influx assay described above. As noted above, such a fusion protein or chemical conjugate could be used as a stimulating antigen or vaccine to generate antibodies to against both LukS-PV and LukF-PV.

**Table 1. Activity of rLukF-PV Mutant Proteins**

| **rLukF-PV** | **Percent Activity as Compared to Wild Type PVL** |
|---|---|
| rLukF-PV (wild type) | 100 |
| rLukF-PVΔI124-S129 | 22.7 |
| rLukF-PV E191A | -1.5 |
| rLukF-PV N173A | 30.9 |
| rLukF-PV R197A | 0.1 |
| rLukF-PV W176A | 2.6 |
| rLukF-PV Y179A | -0.4 |

**Table 2. Activity of rLukS-PV Mutant Proteins**

| **rLukS-PV** | **Percent Activity as Compared to Wild Type PVL** |
|---|---|
| rLukS-PV (wild type) | 100 |
| rLukS-PV ΔD1-17 | 33 |
| rLukS-PV ΔF117-S122 | 49 |
| rLukS-PV T28F | 2 |
| rLukS-PV T28N | 7 |
| rLukS-PV T28D | 9 |
| rLukS-PV T244A | 82 |

### Example 1.0. Polyclonal Antibody Neutralization of PVL Cytotoxic Activities

### Using the Calcium Influx Assay

To determine the neutralizing activity of PVL antibodies, the calcium influx assay was performed as described above, modified in that anti-LukS-PV or anti-LuKF-PV rabbit antiserum was incubated with either rLukS-PV or rLukF-PV 30 min prior to addition to the loaded HL-60 cells. To determine the percent neutralization, the change in fluorescence for this reaction was compared to that of wild type PVL protein alone.

Table 3 shows the neutralization of PVL cytotoxicity determined as described above. Both anti-LukS-PV antiserum and anti-LukF-PV antiserum were effective in neutralizing *in vitro* cytoxicity. When both anti-LukS-PV antiserum and anti-LukF-PV antiserum were evaluated together for neutralization, no synergy was detected. That is, when sub-optimal concentrations of both antibody types were used together (both at 1:160 dilution), greater than additive neutralization was not observed. These results demonstrate that antibodies to only one PVL subunit, e.g., anti-LukF-PV antibodies or anti-LukS-PV antibodies are required for neutralization of PVL toxin.

**Table 3. Neutralization of PVL Cytoxicity by Polyclonal Rabbit Antibodies**

| **PVL Rabbit Anti-Serum** | **Antiserum Dilution** | **% Neutralization** |
|---|---|---|
| None - PVL only control | | 0 |
| Anti-LukS-PV | 1:10 | 107 |
| Anti-LukS-PV | 1:20 | 106 |
| Anti-LukS-PV | 1:40 | 108 |
| Anti-LukS-PV | 1:80 | 105 |
| Anti-LukS-PV | 1:160 | -7 |
| Anti-LukS-PV | 1:320 | 12 |
| Anti-LukF-PV | 1:10 | 114 |
| Anti-LukF-PV | 1:20 | 118 |
| Anti-LukF-PV | 1:40 | 114 |
| Anti-LukF-PV | 1:80 | 114 |
| Anti-LukF-PV | 1:160 | 26 |
| Anti-LukF-PV | 1:320 | 14 |
| Anti-LukS-PV Anti-LukF-PV | 1:160 1:80 | >100 |
| Anti-LukS-PV Anti-LukF-PV | 1:160 1:160 | 0 |

### Example 11. In Vitro Determination of PVL Cytotoxicity by XTT Assay

A solution containing rLukS-PV and rLukF-PV (20 nM each) was prepared in high-glucose Dulbecco's modified Eagle's medium without phenol red (HG-DMEM) (Gibco), supplemented with 50 µg/mL gentamicin and 1% heat inactivated fetal bovine serum (HI-FBS) (Gibco), maintenance medium (MM). Serial 2-fold dilutions of toxin from 20 nM were performed in MM on a 96-well cell culture plate. Negative medium control wells and cell control wells, each set containing MM instead of diluted toxin, were included on every assay plate. Approximately 5 x 10⁵ viable HL-60 cells (induced with dimethylsulfoxide (DMSO) to differentiation to more mature and PVL susceptible cells of the neutrophilic pathway) were added to each well with diluted toxin and medium for cell control. All media control wells received MM instead of a cell suspension. The assay plate with samples was incubated under humidified conditions in a 37°C incubator with 8% CO₂ for 24-48 hours.

XTT as the sodium salt of the compound 2,3-bis[2-methoxy-4-nitro-5-sulfophenyl]-2H-tetrazolium-5-carboxyanilide (Sigma, cat. # TOX-2), and prepared in MM according to manufacturer's instructions, was then added to all wells at 20% of culture medium volume in each well. The plate was returned to the incubator for additional incubation to allow for a color development due to XTT action. (Mitochondrial dehydrogenases of living cells cleave the tetrazolium ring of XTT, resulting in a solution of orange color being developed.) The plate was then removed from the incubator, centrifuged to pellet cells and debris before a volume of the supernatant from each well was transferred to corresponding wells of a round bottom ELISA plate. Optical densities (OD) of the supernatants were measured at 450 nm with the aid of an ELISA plate reader that subtracts medium only OD as background before reporting data. The percent of cells that were killed due to PVL cytotoxic action was then calculated.

As determined by the XTT assay, both wild type PVL subunits, rLukF-PV and rLukS-PV at ≥ 0.5 nM, are required for *in vitro* cytoxicity. No cytotoxicity was observed for each PVL subunit alone at 10 nM. Thus data further demonstrates that one PVL subunit alone is not cytoxic and can be individually as a vaccine or stimulating antigen.

### Example 12. Antibody Neutralization of PVL Cytotoxicity Using the XTT Assay

Purified antibodies from mouse immune sera collected from the LukS-PV and LukF-PV studies performed to generate immunized splenocytes for hybridoma production in mammalian cell fusion experiments and, from ascites fluid generated with established hybridomas secreting MAbs specif for toxin sub-units, were characterized for their capacities to neutralize PVL toxin *in vitro.*

Serial 2-fold dilutions of the antibodies were performed on a 96-well cell culture plate. Negative medium control wells and cell control wells, each containing no toxin, and a set of positive toxin control wells were included on every assay plate. An equal volume of 40 nM PVL toxin subunits in MM (described above) was added to all wells with antibody and those for toxin positive control. MM at equal volume was added to all medium control wells and cell control wells. To each well with diluted antibody, toxin and medium for cell control, was added approximately 1 x 10⁶ viable DMSO induced HL-60 cells in a volume equal to that in each well. All media control wells received MM instead of a cell suspension. All antibody and toxin concentrations were thus diluted 4 times that of starting concentrations. The content of each well was mixed and a 50% volume was transferred to corresponding well of another 96-well cell culture plate. Both plates were incubated in a humidified 37°C incubator with 8% CO₂.

XTT as the sodium salt of the compound 2,3-bis[2-methoxy-4-nitro-5-sulfophenyl]-2H-tetrazolium-5-carboxyanilide (Sigma, cat. # TOX-2), and prepared in MM according to manufacturer's instructions, was then added to all wells at 20% of culture medium volume in each well. The plate was returned to the incubator for additional incubation to allow for a color development due to XTT action. The plate was then removed from the incubator, centrifuged to pellet cells and debris before a volume of the supernatant from each well was transferred to corresponding wells of a round bottom ELISA plate. Optical densities (OD) of the supernatants were measured at 450 nm. The percent of cells that were killed due to PVL cytotoxic action was then calculated.

As set forth in Table 4, MAbs to LukS-PV were 10 times more effective at neutralizing PVL cytoxocity than MAbs to LukF-PV in this assay. That is, approximately ten-fold higher LukF MAb was required for neutralization of PVL cytotoxicity *in vitro* as determined by the XTT assay. For example, 50% neutralization of PVL cytotoxicity was achieved using 0.4-10 µg/mL LukS MAb, whereas 5-95 µg/mL LukF Mab was required to achieve 50% neutralization. The results in Table 4 also show that polyclonal antibodies specific to rLukS-PV ("LukS-M-IgG") or rLukF-PV ("LukF-M-IgG") were able to neutralize PVL cytoxicity *in vitro.*

These results indicate that a composition comprising a LukS-PV antigen alone (i.e., without LukF-PV) would be effective as a vaccine, and that a composition comprising anti-LukS-PV antibody (including MAb, or IVIG or hyperimmune specific IVIG comprising anti-LukS-PV antibodies) alone (i.e., without anti-LukF-PV antibody) would be effective for neutralizing PVL cytoxicity. Although comparable LukF-PV antigen/antibody compositions might be less potent, they also would be useful, as demonstrated by the ability of LukF Mab alone to neutralize PVL cytotoxicity in this assay.

**Table 4. MAb 50% Neutralization of PVL Cytotoxic Killing (XTT Assay)**

| **Mab** | **Mab Specificity** | **Concentration for 50% Neutralization (**µ**g/mL)** |
|---|---|---|
| 1LukS142 | rLukS-PV | 0.8 |
| 1LukS166 | rLukS-PV | 0.4 |
| 1LukS235 | rLukS-PV | 0.7 |
| 1LukS276 | rLukS-PV | 9.8 |
| 1LukS500 | rLukS-PV | 0.6 |
| 1LukS633 | rLukS-PV | 1.8 |
| 1LukF259 | rLukF-PV | 25.1 |
| 1LukF343 | rLukF-PV | 5.7 |
| 1LukF408 | rLukF-PV | 6.6 |
| 1LukF438 | rLukF-PV | 11.5 |
| 1LukF823 | rLukF-PV | 44.9 |
| 1LukF951 | rLukF-PV | 94.6 |

### Example 13. Neutralization of PVL-Mediated Cytotoxicity By

### Monoclonal or Polyclonal anti-LukS Antibodies

Peripheral blood was drawn from healthy volunteers and human PMN were purified by a Percoll gradient. Monoclonal (1LukS235) or polyclonal (rabbit) anti-LukS-PV antibody was added at different dilutions to human PMNs (5x10⁵ cells/well) to inhibit the cytotoxic effect of rPVL (10 nM) or USA300 24 hr culture supernatant (1:40 dilution), which contained LukS-PV at 1.2 µg/ml and LukF-PV at 0.5 µg/ml. These selected dilutions induced respectively 85%(MAb) and 70% (PAb) of cytotoxicity on human PMNs. As controls, rabbit serum and anti-alpha toxin MAb were utilized. The inhibitory effect of the anti-LukS-PV antibodies was evaluated by an XTT assay after 2 hours of culture, as measured by the change of fluorescence at 450 nm.

The results indicated that induced HL-60 cells and peripheral blood purified human PMNs were susceptible to rPVL at the same concentrations. rPVL and PVL obtained from a culture supernatant of CA-MRSA USA300 were similarly effective in inducing cytotoxicity on human PMNs. As demonstrated in Table 5, both polyclonal and monoclonal antibodies were effective in neutralizing PVL-dependent cytotoxicity on human PMNs. As control, naïve rabbit serum (1:10 dilution) only induced 10% neutralization of cytotoxicity for both rPVL and PVL containing USA300 supernatant. These results demonstrate that monoclonal and polyclonal anti-LukS-PV antibodies are effective at neutralizing the cytotoxic effects of both rPVL and native PVL expressed by CA-MRSA.

**Table 5. MAb & PAb Neutralization of PVL Cytoxicity (XTT Assay)**

| **Anti-LukS-PV Rabbit Serum Dilution** | **% Cytotoxic Neutralization of rPVL (10 nM)** | **% Cytotoxic Neutralization of PYL containing USA300 Supernatant (1:40)** |
|---|---|---|
| none | 0 | 0 |
| 1:10 | 82 | 82 |
| 1:20 | 93 | 97 |
| 1:40 | 64 | 64 |
| 1:80 | 11 | 11 |
| 1:160 | 12 | 12 |
| 1:320 | 14 | 13 |

| **Mab 1LukS235 Dilution (**µ**g/ml)** | **% Cytotoxic Neutralization of rPVL (10 nM)** | **% Cytotoxic Neutralization of PVL containing USA300 Supernatant (1:40)** |
|---|---|---|
| none | 0 | 0 |
| 40 | 93 | 44 |
| 20 | 89 | 33 |
| 10 | 95 | 35 |
| 5 | 82 | 30 |
| 2.5 | 25 | 28 |
| 1.25 | 14 | 34 |

### Example 14. Taxicity of the PVL Toxin, rLukF-PV and rLukS-PV

New Zealand female rabbits (Harlan), 4-5 month old, were shaved and injected intradermally with increasing doses of rLukF-PV and rLukS-PV, or rPVL toxin (rLukF-PV and rLukS-PV) at roughly equimolar concentrations (12.5, 25, 50 and 100 µg). Dermonecrosis was followed daily for 1 week; size of the lesions was measured.

This study demonstrated the susceptibility of rabbits to dermonecrosis caused by intradermal injection of rPVL toxin (rLukF-PV and rLukS-PV). Although dermonecrosis was observed after 24 h when rPVL toxin (12.5-100 µg) was injected, injection of a single PVL subunit (either 200 µg rLukS-PV or 200 µg rLukF-P) did not produce any lesions. A dose-dependent effect was observed where the size of the lesion increased with increasing concentrations of rPVL toxin. These results further demonstrate that either rLukS-PV or rLukF-PV alone is a non toxic antigen that can be used as a vaccine or stimulation antigen without further detoxification.

### Example 15. Efficacy of PVL Antibodies in Neutralizing PVL Toxin In Vivo

The ability of vaccines comprising rLukS-PV, rLukF-PV, or rPVL (rLukS-PV + rLukF-PV) to neutralize rPVL toxin (12.5-200 µg) *in vivo* was assessed. New Zealand female rabbits, 5-6 month old, were immunized 3 x 2 weeks apart via intramuscular route with 50 µg of rLukS-PV, 50 µg rLukF-PV, or with both subunits (50 µg of each), utilizing Titermax (Sigma) as an adjuvant in a 1:1 ratio. Rabbits were bled seven days after the third injection and the IgG titers for LukS-PV and LukF-PV were evaluated by ELISA. In all relevant sera, titers for LukS-PV IgG, and LukF-PV IgG were 1/10⁶ dilution for an OD₄₅₀ₙₘ = 2.0. Antisera from rabbits immunized with rLukF-PV reacted only with rLukF-PV, while rabbits immunized with rLukS-PV reacted with only rLukS-PV, demonstrating that there is no cross-reactivity between the heterologous subunits and antibodies.

Rabbits were shaved and injected (challenged) on their back with rPVL toxin (200 µg each subunit), 200 µg rLukF-PV, 200 µg rLukS-PV or PBS. Vaccination with rLukS-PV and rLukF-PV induced high antibody titers for each subunit, respectively (dilution 1/10⁶ for OD = 2). Moreover, these antibodies demonstrated protection against dermonecrosis resulting from the rPVL toxin challenge. That is, post-rPVL challenge, no dermonecrosis was observed in rabbits immunized with either rPVL subunit (rLukS-PV or rLukF-PV). In contrast, dermonecrosis was observed on control rabbits, which received placebo (PBS plus Titermax). Additionally, in all rabbits, no necrosis was observed when only one PVL subunit was used as the challenge.

These results further demonstrate that immunization with rLukS-PV or rLukF-PV alone (i.e., without the other subunit) is effective in preventing necrosis caused by PVL. Thus, a composition comprising one of these PVL antigens would be useful for the prevention of necrosis caused by PVL producing CA-MRSA.

### Example 16. Efficacy of PVL Antibodies in Protection Against

### PVL+CA-MRSA Infection

The ability of vaccines comprising rLukS-PV, rLukF-PV, or rPVL (rLukS-PV + rLukF-PV) to neutralize PVL producing *S. aureus* skin infections was assessed. New Zealand female rabbits, 5-6 month old, were immunized 3 x 2 weeks apart via intramuscular route with 50 µg of rLukS-PV, 50 µg rLukF-PV, or with both subunits (50 µg of each), utilizing Titermax (Sigma) as an adjuvant in a 1:1 ratio. Rabbits were bled seven days after the third injection and the LukS-PV and LukF-PV IgG titers were evaluated by ELISA. In all relevant sera, titers for LukS-PV IgG, and LukF-PV IgG were 1/10⁶ dilution for an OD₄₅₀ₙₘ = 2.0. Antisera from rabbits immunized with rLukF-PV reacted only with rLukF-PV, while rabbits immunized with rLukS-PV reacted with only rLukS-PV, demonstrating that there is no cross-reactivity between the heterologous subunits and antibodies. Antisera from rabbits immunized with both subunits reacted with both LukF-PV and LukS-PV.

Rabbits were shaved and injected on their back with 10⁸ CFU/100 µL of *S*. *aureus* strains, USA300 (PVL producing CA-MRSA. Vaccination with rLukS-PV and rLukF-PV induced high antibody titers for each subunit, respectively (dilution 1/10⁶ for OD = 2). These antibodies showed protection against dermonecrosis resulting from a PVL producing *S. aureus* isolate (or CA-MRSA USA300). That is, no dermonecrosis was observed on rabbits immunized with either rPVL subunit (rLukS-PV or rLukF-PV) or with rPVL (rLukS-PV and rLukF-PV). In contrast, dermonecrosis was observed on control rabbits, which received placebo (PBS plus Titermax) or naïve rabbit. In addition, rabbits that were immunized with rPVL (rLukS-PV and rLukF-PV) had a reduced severity of infection. None of the rPVL-immunized rabbits developed lesions, whereas the control rabbit did produce lesions. Rabbits immunized with either rLukS-PV or rPVL (rLukF-PV and rLukS-PV) were healthy on day 7. However, the rabbit immunized with rLukF-PV demonstrated clinical signs of morbidity (weight loss, fever) on day 5 and the control rabbit, which received PBS, died after 40 hr. The rLukS-PV vaccine and the rPVL (rLukF-PV and rLukS-PV) vaccine were effective in preventing CA-MRSA infections. The morbidity signs in the single rabbit immunized with the rLukF-PV vaccine may indicate that the rLukF-PV vaccine was not as effective as the rLukS-PV vaccine, but the sample size is too small to draw a scientifically valid conclusion. In any event, the rLukF-PV vaccine at least delayed the onset and/or severity of the disease, even if the rabbit was not full protected from infection.

### Example 17. Cloning, Expression, and Purification of PVL Antigens

Nucleotide sequences encoding LukF-PV and LukS-PV are cloned by the polymerase chain reaction (PCR) from *S*. *aureus* ATCC No. 49774 genomic DNA into a pTrcHis-B vector (Stratagene) using Nco I and BamH1 restriction sites at the amino and carboxy termini, respectively. Plasmids pTrcLukS PV1 and pTrcLukF PV1 are formed and confirmed by DNA sequencing. Expression of the plasmid is under control of a lac operon and therefore, IPTG is used for inducing protein expression. Expression is effected in *E.coli* cells transformed with the plasmids.

The LukF-PV and LukS-PV subunits are then purified from *E. coli* cells using a two-step column scheme. *E. coli* cells containing LukF-PV and LukS-PV were lysed and cell debris was removed by centrifugation. The cell lysate was first loaded onto a SP-Sepharose column in 0.05M NaCl/0.02 M sodium phosphate, pH 6.5 containing 5% glycerol, and eluted with a 0.05-0.5 M NaCl linear gradient. Fractions that contained antigen, LukF-PV or LukS-PV, as detected by SDS-PAGE, were pooled. The pooled antigen was further purified on a ceramic hydroxyapatite (CHT) affinity column. The CHT column was first equilibrated with 0.05 M NaCl/0.02 M sodium phosphate, pH 6.8 containing 5%glycerol and eluted with a linear gradient of 0.05M-0.75 M NaCl. The final products were analyzed for purity using SDS-PAGE/silver staining.

### Example 18. Generating Non-Toxic PVL Mutant Proteins

Non-toxic PVL mutant proteins are generated by mutagenesis. The mutant proteins are generated by PCR cloning/mutagenesis techniques. Mutants are created using plasmid DNA [pTrcLukF PV1 (for LukF-PV) and pTrcLukS PV1(for LukS-PV)] as a template DNA using standard site directed mutagenesis methods. The mutant proteins contain mutations that eliminate the ability of the LukS-PV subunit to insert itself into a cell membrane, prevent stem or cytoplasmic extremity of a transmembrane domain from unfolding for LukS or F, alter the phosphorylation site that is required for leukocytolytic activity, block Ca⁺2 channel activity, block activity of PVL pore, and/or disrupt the interaction between the LukS-PV and LukF-PV subunits.

### Example 19. Neutralization Assays

The antisera from animal studies is assayed by ELISA for titers against the PVL LukF-PV and LukS-PV subunits. Antisera with high titers are used in a neutralization assay. Essentially, the cytotoxicity assays are performed by adding a neutralizing antibody. The antibody is added to the PVL subunits prior to mixing with the polymorphonuclear cells or added simultaneously to the polymorphonuclear cells polymorphonuclear cells during the assay. Neutralization is detected by observing a reduction of the morphological changes caused by PVL and/or detecting a decrease in the fluorescent emissions caused by the influx of cations through either the activated calcium channels or PVL pores.

### Example 20. PVL Antibody Production

### Donor stimulation

PVL proteins are used to vaccinate plasma donors. Plasma is collected and IgGs purified using standard methodology.

### Generation of PVL specific monoclonal antibodies (MAbs)

For MAb production, four groups of BALB/c mice are immunized with a PVL toxin (either a LukF-PV subunit, a LukS-PV subunit, or both) at two groups of mice per toxin. Immunizations are performed at two-week intervals with the exception of the final injection that is given 3 days prior to sacrificing the mice. Each toxin is injected at 5µg and 10µg per mouse of the respective groups. The toxins are administered in combination with complete and incomplete Freund's adjuvant sequentially, for the first two injections. Subsequent injections are performed with toxin diluted in phosphate buffered saline (PBS). Splenocytes suspensions are prepared as a pool of the respective groups from the sacrificed mice and stored in appropriate aliquots in liquid nitrogen for use in fusion experiments at future dates. An inventory of concentrated stock supplies of MAbs specific to LukF-PV and LukS-PV are produced in approximately 4 months of the dates of fusion experiments.

After the mouse monoclonal antibodies are selected, they are humanized by splicing the mouse genes for the highly specific antigen-recognizing portion of the antibody into the human genes that encode the rest of the antibody molecule. The humanized monoclonal antibodies typically contain less than 10% mouse content, thus minimizing any immune reaction.

### Recombinant Antibody Production - Generation of Antibodies using Phage Display

Two strategies are applied when using phage display technology. The first is the use of ready-made libraries known as naive libraries that express either peptides or human antibody fragments. The second involves construction of an antibody library that is specific for the protein of interest.

Before determining which approach is used, commercial libraries are researched and compared. Alternatively, spleen cells are used from an immunized animal to construct a library specific for the protein of interest.

After the library strategy is decided, LukS-PV and LukF-PV are used as targets for screening. The LukS-PV and LukF-PV subunits are adsorbed to a solid support for capturing the phage displaying the specific peptides or antibody fragments. The captured phage particles are eluted differentially for the selection of higher affinity peptides or antibody fragments. Three rounds of selection are generally used for isolation of highly specific peptides or antibody fragments. Random mutagenesis of the phage libraries are also be used to enhance specificity, if required.

Various substitutions and modifications may be made to the invention disclosed herein without departing from the scope and spirit of the invention. The foregoing description and examples are illustrative only, and do not limit the scope of the invention, which is defined by the claims.

## Claims

1. A composition comprising a PVL antigen of *S. aureus* and a pharmaceutically acceptable carrier.

2. The composition of claim 1, wherein the PVL antigen is conjugated to another bacterial antigen.

3. The composition of claim 2, wherein the other bacterial antigen is selected from the group consisting of *S*. *aureus* Type 5, *S*. *aureus* Type 8, *S*. *aureus* 336, *S*. *epidermidis* PS1, *S. epidermidis* GP1, α-toxin, lipoteichoic acid (LTA) and microbial surface components recognizing adhesive matrix molecule (MSCRAMM) proteins.

4. The composition of claim 2, comprising a PVL antigen conjugate selected from the group consisting of (a) a LukF-PV subunit conjugated to a LukS-PV subunit; (b) a LukF-PV subunit conjugated to another LukF-PV subunit; and (c) a LukS-PV subunit conjugated to another LukS-PV subunit.

5. The composition of claim 1, wherein the PVL antigen comprises a mutation in at least one of the LukF-PV or LukS-PV amino acid sequence, relative to its wild-type sequence, comprising at least one amino acid substitution, insertion, or deletion.

6. The composition of claim 5, wherein the PVL antigen comprises at least one mutation selected from the group consisting of mutations that (i) prevent PVL binding to a cell membrane, (ii) prevent a stem or cytoplasmic extremity of a transmembrane domain from unfolding for LukS or F, (iii) block assembly of LukF-PV and LukS-PV, (iv) block Ca⁺² channel activity, (v) block activity of a PVL pore, (vi) alter the phosphorylation site of LukS-PV, (vii) disrupt membrane binding cleft of LukF-PV; (viii) create N-terminal deletions of the "amino latch" of PVL antigens, and (ix) create cysteine double mutants that prevent unfolding of pre-stem and insertion into the membrane.

7. The composition of claim 5, wherein the composition comprises PVL antigen selected from the group consisting of (a) PVL antigen comprising a mutated LukF-PV subunit and a wild-type LukS-PV subunit; (b) PVL antigen comprising a wild-type LukF-PV subunit and a mutated LukS-PV subunit and (c) PVL antigen comprising a mutated LukF-PV subunit and a mutated LukS-PV subunit.

8. The composition of claim 1, wherein the composition comprises a LukF-PV subunit and no LukS-PV subunit or a LukS-PV subunit and no LukF-PV subunit.

9. The composition of claim 1, further comprising one or more additional bacterial antigens.

10. The composition of claim 9, wherein said one or more additional bacterial antigens is selected from the group consisting of *S*. *aureus* Type 5, *S. aureus* Type 8 and S. *aureus* 336, *S*. *epidermidis* PS1, *S*. *epidermidis* GP1, α-toxin, lipoteichoic acid (LTA) and microbial surface components recognizing adhesive matrix molecule (MSCRAMM) proteins.

11. The composition of claim 9, further comprising one or more additional PVL antigens.

12. A PVL antigen comprising a Panton-Valentine Leukocidin (PVL) antigen of *S*. *aureus* conjugated to another bacterial antigen.

13. The PVL antigen of claim 12, wherein the PVL antigen is selected from the group consisting of purified wild-type PVL antigens and recombinant PVL antigens.

14. The PVL antigen of claim 12, wherein the other bacterial antigen is selected from the group consisting of *S*. *aureus* Type 5, *S*. *aureus* Type 8, *S*. *aureus* 336, *S*. *epidermidis* PS1, *S*. *epidermidis* GP1, α-toxin, lipoteichoic acid (LTA) and microbial surface components recognizing adhesive matrix molecule (MSCRAMM) proteins.

15. The PVL antigen of claim 12, comprising a conjugate selected from the group consisting of (i) a LukF-PV subunit conjugated to a LukS-PV subunit; (ii) a LukF-PV subunit conjugated to another LukF-PV subunit; and (iii) a LukS-PV subunit conjugated to another LukS-PV subunit.

16. The PVL antigen of claim 12, selected from the group consisting of (a) PVL antigen comprising a LukF-PV subunit and no LukS-PV subunit and (b) PVL antigen comprising a LukS-PV subunit and no LukF-PV subunit.
